# EUROPEAN PATENT APPLICATION

(11) **EP 4 737 563 A1**
(43) Date of publication of application: **06.05.2026**
(21) Application number: 23943445.9
(22) Date of filing: 20.12.2023
(51) Int. Cl.: C12N 7/01, C12N 15/12, A61K 35/768, A61K 38/17, A61P 35/00, C12R 1/93

(54) **RECOMBINANT ONCOLYTIC VACCINIA VIRUS AND USE THEREOF**

(30) Priority: 29.06.2023 CN 202310781871
(71) Applicant: Suzhou Onlyv Biotechnology Limited Company, Suzhou, Jiangsu 215131 (CN)
(72) Inventor: JU, Songguang, Suzhou, Jiangsu 215131 (CN); GE, Yan, Suzhou, Jiangsu 215131 (CN)
(74) Representative: AWA Denmark A/S
(86) International application number: PCT/CN2023/140367
(87) International publication number: WO 2025/000972

(57) **Abstract**

Provided in the present invention is a recombinant oncolytic vaccinia virus, which is operably inserted into a synonymously mutated exogenous gene capable of expressing 4-1BBL, and also provided is the use of the recombinant oncolytic vaccinia virus in the preparation of a drug for preventing or treating tumors and cancers. The present invention has the following beneficial effects: the synonymous-mutation-based recombinant vaccinia virus VV-mH4-1BBL retains the original oncolytic effect of the oncolytic virus and the functions thereof of initiating and enhancing anti-tumor immune responses, and improves the safety by means of deleting the TK gene; 4-1BBL is highly expressed on the surface of a tumor cell, such that 4-1BBL can enhance anti-tumor immunity by means of exciting a 4-1BB signal of 4-1BB + immune cells (including T cells) in the tumor microenvironment, and 4-1BBL is also confined within tumor tissues to exert the function thereof in a centralized manner, thereby avoiding potential systemic toxic side effects; and the introduction of a synonymous mutation site enables the virus to detect the expression of a therapeutic (exogenous) 4-1BBL gene during treatment.

## Description

### Technical Field

The invention relates to the technical field of biomedicine, in particular to a recombinant oncolytic vaccinia virus and use thereof.

### Background

Stimulation of 4-1BB signaling in immune cells can elicit strong and sustained anti-tumor immunity. At present, 4-1BB agonistic monoclonal antibodies, such as urelumab and utomilumab, have entered clinical research. When these antibodies are used systemically, because of the small difference between the effective dose and the safe dose, systemic toxic side effects often occur when the anti-tumor immune effect is effectively stimulated, or the anti-tumor effect is not significant when there are no obvious toxic side effects. These contradictions greatly limit their clinical application.

Soluble 4-1BBL can stimulate 4-1BB signal, but is not used in clinical application studies. If it is directly used systemically, it can strongly enhance the immune response in the whole body, resulting in serious toxic side effects.

Oncolytic viruses can selectively infect tumor cells, induce immunogenic death of tumor cells, initiate anti-tumor immunity and cause local inflammation, and act as immune adjuvants. However, oncolytic virus alone is often used to treat tumors because the antiviral effect of the body can quickly eliminate the virus, so it is necessary to enhance the anti-tumor immunity triggered by oncolytic virus after it plays its role in order to induce strong and sustained anti-tumor effects. Therefore, it is necessary to introduce immune enhancing factors to improve the anti-tumor effect of oncolytic viruses.

It has been reported that mouse 4-1BBL recombinant oncolytic virus was constructed by using mouse 4-1BBL gene, and its reliability was proved by mouse model. However, recent studies have found that the wild-type 4-1BBL gene is commonly expressed in tumor cells such as intestinal cancer, and its encoding product (4-1BBL molecule) is located in the cells but not on the surface the cells. Therefore, if the wild-type 4-1BBL gene is used to construct a 4-1BBL recombinant oncolytic virus for treatment, the expression and localization of therapeutic 4-1BBL will not be detected and tracked.

Oncolytic virus can selectively infect and cause immunogenic death of tumor cells, and then trigger the body's anti-tumor immune response, making it a feasible means of tumor immunotherapy, thereby to enter the field of tumor immune drug research and development. However, oncolytic virus itself is easy to be cleared by the body, and the anti-tumor immunity triggered by oncolytic virus is facing the common problem of being blocked by negative mechanisms such as immune checkpoints. Therefore, the overall design idea in the field is to construct recombinant oncolytic viruses, retain the advantages of the original oncolytic virus, avoid the weaknesses thereof, and then introduce immune enhancing factors to overcome the negative immune mechanism and induce safe, effective, systemic and sustained anti-tumor immunity.

Costimulatory molecule ligand 4-1BBL strongly promotes the body's anti-tumor immunity by acting on 4-1BB molecules on the surface of T cells, NK cells, DC and so on, that is, by 4-1BB/4-1BBL pathway. Therefore, the biological agents taking 4-1BB/4-1BBL pathway as a target may become a new type of anti-tumor drugs. Agonistic 4-1BB monoclonal antibody and 4-1BBL recombinant oncolytic virus are important biological agents in the field, but their shortcomings restrict their future application and need to be improved.

The agonistic 4-1BB monoclonal antibody can activate the 4-1BB/4-1BBL pathway to produce strong anti-tumor effect, but the dosage difference between the effective anti-tumor effect and the toxic side effect of the agonistic 4-1BB monoclonal antibody is small, that is, the safe dosage range is small. Thus, the selection of the natural ligand 4-1BBL as an immune activator of the 4-1BB molecule provides a natural stimulus to activate the 4-1BB signal. In addition, if soluble 4-1BBL is directly used to treat tumors, it may bring toxic side effects due to the systemic effects of cytokines.

However, it has been reported that 4-1BBL molecules generally exist in intestinal cancer, pancreatic cancer and other tumor cells, but are not expressed in the cell membrane, so that tumor cells can avoid stimulating 4-1BB molecules on the surface of immune cells, which is a mechanism of tumor immune escape. If the natural ligand 4-1BBL is used to construct a recombinant oncolytic virus, 4-1BBL can be expressed on the surface of tumor cells to stimulate anti-tumor immunity. However, in the future treatment process, it is impossible to distinguish whether the 4-1BBL gene in the tumor tissue comes from the tumor tissue itself or the therapeutic 4-1BBL recombinant oncolytic virus, which makes it extremely difficult to monitor and evaluate the efficacy.

### Summary of the Invention

Aiming at the existing technical limitation as mentioned above, the invention provides a recombinant oncolytic vaccinia virus and the use thereof, establishes a novel 4-1BB signal stimulation mode, maintains the immune effect of 4-1BBL in stimulating the 4-1BB signal, improves the safety, and provides a novel biological preparation capable of detecting and monitoring the treatment process and the efficacy, overcoming the deficiencies and drawbacks mentioned in the background.

In order to achieve the above purposes, the invention adopts the following technical solutions:
The invention point of the invention is to provide a recombinant oncolytic vaccinia virus which is operably inserted into a synonymously mutated exogenous gene capable of expressing 4-1BBL.

Alternatively, according to the above recombinant oncolytic vaccinia virus, the exogenous gene is inserted into TK gene of the vaccinia virus.

Alternatively, according to the above recombinant oncolytic vaccinia virus, DNA sequence of the exogenous gene is shown as SEQ ID No.1 to SEQ ID No.3.

The applicant has listed three synonymously mutated gene sequences, which do not limit the scope of the invention. In fact, as long as the polypeptide/amino acid/protein sequences finally obtained through encoding are the same (same or similar efficacy), the synonymous mutation at any one or more sites is feasible.

The sequence of SEQ ID No.1 (mutation of 540T to C) is:

atggaatacgcctctgacgcttcactggaccccgaagccccgtggcctcccgcgccccgcgctcgcgcctgccgcgtactgccttgg
gccctggtcgcggggctgctgctgctgctgctcgctgccgcctgcgccgtcttcctcgcctgcccctgggccgtgtccggggctcgcgc
ctcgcccggctccgcggccagcccgagactccgcgagggtcccgagctttcgcccgacgatcccgccggcctcttggacctgcggcaggg
catgtttgcgcagctggtggcccaaaatgttctgctgatcgatgggcccctgagctggtacagtgacccaggcctggcaggcgtgtccctgac
gggggggcctgagctacaaagaggacacgaaggagctggtggtggccaaggctggagtctactatgtcttctttcaactagagctgcggcgcg
tggtggccggcgagggctcaggctccgtttcacttgcgctgcacctgcagccactgcgctctgctgctggggccgccgccctggcCttgacc
gtggacctgccacccgcctcctccgaggctcggaactcggccttcggtttccagggccgcttgctgcacctgagtgccggccagcgcctggg
cgtccatcttcacactgaggccagggcacgccatgcctggcagcttacccagggcgccacagtcttgggactcttccgggtgacccccgaaa
tcccagccggactcccttcaccgaggtcggaataa.

The sequence of SEQ ID No.2 (mutation of 468G to A) is:

atggaatacgcctctgacgcttcactggaccccgaagccccgtggcctcccgcgccccgcgctcgcgcctgccgcgtactgccttgg
gccctggtcgcggggctgctgctgctgctgctcgctgccgcctgcgccgtcttcctcgcctgccccctgggccgtgtccggggctcgcgc
ctcgcccggctccgcggccagcccgagactccgcgagggtcccgagctttcgcccgacgatcccgccggcctcttggacctgcggcaggg

catgtttgcgcagctggtggcccaaaatgttctgctgatcgatgggcccctgagctggtacagtgacccaggcctggcaggcgtgtccctgac
ggggggcctgagctacaaagaggacacgaaggagctggtggtggccaaggctggagtctactatgtcttctttcaactagagctgcggcgcg
tggtggccggcgaAggctcaggctccgtttcacttgcgctgcacctgcagccactgcgctctgctgctggggccgccgccctggctttgacc
gtggacctgccacccgcctcctccgaggctcggaactcggccttcggtttccagggccgcttgctgcacctgagtgccggccagcgcctggg
cgtccatcttcacactgaggccagggcacgccatgcctggcagcttacccagggcgccacagtcttgggactcttccgggtgacccccgaaa
tcccagccggactcccttcaccgaggtcggaataa.

The sequence of SEQ ID No.3 (mutation of 597C to T) is:

atggaatacgcctctgacgcttcactggaccccgaagccccgtggcctcccgcgccccgcgctcgcgcctgccgcgtactgccttgg
gccctggtcgcggggctgctgctgctgctgctcgctgccgcctgcgccgtcttcctcgcctgcccctgggccgtgtccggggctcgcgc
ctcgcccggctccgcggccagcccgagactccgcgagggtcccgagctttcgcccgacgatcccgccggcctcttggacctgcggcaggg
catgtttgcgcagctggtggcccaaaatgttctgctgatcgatgggcccctgagctggtacagtgacccaggcctggcaggcgtgtccctgac
gggggggcctgagctacaaagaggacacgaaggagctggtggtggccaaggctggagtctactatgtcttctttcaactagagctgcggcgcg
tggtggccggcgagggctcaggctccgtttcacttgcgctgcacctgcagccactgcgctctgctgctggggccgccgccctggctttgaccg
tggacctgccacccgcctcctccgaggctcggaactcggccttcggtttTcagggccgcttgctgcacctgagtgccggccagcgcctggg
cgtccatcttcacactgaggccagggcacgccatgcctggcagcttacccagggcgccacagtcttgggactcttccgggtgacccccgaaa
tcccagccggactcccttcaccgaggtcggaataa.

Alternatively, according to the above recombinant oncolytic vaccinia virus, amino acid sequence of the exogenous gene is shown as SEQ ID No.4.

The sequence of the SEQ ID No.4 is:

MEYASDASLDPEAPWPPAPRARACRVLPWALVAGLLLLLLLAAACAVFLACPWAVSG
ARASPGSAASPRLREGPELSPDDPAGLLDLRQGMFAQLVAQNVLLIDGPLSWYSDPGLAG
VSLTGGLSYKEDTKELVVAKAGVYYVFFQLELRRVVAGEGSGSVSLALHLQPLRSAAGAA
ALALTVDLPPASSEARNSAFGFQGRLLHLSAGQRLGVHLHTEARARHAWQLTQGATVLGL
FRVTPEIPAGLPSPRSE.

Alternatively, according to the above recombinant oncolytic vaccinia virus, the exogenous gene is linked via an IRES sequence.

IRES is a ribosome entry site and is only one of the linker sequences that can be used.

Alternatively, according to the above recombinant oncolytic vaccinia virus, the IRES sequence is shown as SEQ ID No.5.

The sequence of the SEQ ID No.5 is:

TTATCATCGTGTTTTTCAAAGGAAAACCACGTCCCCGTGGTTCGGGGGGCCTAGAC
GTTTTTTAACCTCGACTAAACACATGTAAAGCATGTGCACCGAGGCCCCAGATCAGATC
CCATACAATGGGGTACCTTCTGGGCATCCTTCAGCCCCTTGTTGAATACGCTTGAGGTG

AGCCATTTGACTCTTTCCACAACTATCCAACTCACAACGTGGCACTGGGGTTGTGCCGC
CTTTGCAGGTGTATCTTATACACGTGGCTTTTGGCCGCAGAGGCACCTGTCGCCAGGTG
GGGGGTTCCGCTGCCTGCAAAGGGTCGCTACAGACGTTGTTTGTCTTCAAGAAGCTTC
CAGAGGAACTGCTTCCTTCACGACATTCAACAGACCTTGCATTCCTTTGGCGAGAGGG
GAAAGACCCCTAGGAATGCTCGTCAAGAAGACAGGGCCAGGTTTCCGGGCCCTCACAT
TGCCAAAAGACGGCAATATGGTGGAAAATAACATATAGACAAACGCACACCGGCCTTAT
TCCAAGCGGCTTCGGCCAGTAACGTTAGGGGGGGGGGAGGGAGAGGGG.

The second invention point of the invention is to provide a pharmaceutical composition, which comprises the recombinant oncolytic vaccinia virus, a pharmaceutically acceptable carrier and a pharmaceutical excipient.

Alternatively, according to the above pharmaceutical composition, the administration mode of the pharmaceutical composition is direct injection and/or injection in combination with an endoscope; and the endoscope is preferably a laparoscope, a choledochoscope, a thoracoscope, an enteroscope, and a neuroendoscope.

The third invention point of the invention is to provide use of the recombinant oncolytic vaccinia virus or the pharmaceutical composition in the preparation of a drug for preventing or treating a tumor and/or a cancer.

Alternatively, according to the above use, the tumor and/or cancer is a solid tumor, preferably a cold tumor and/or a hot tumor, the histological types thereof include, but are not limited to, pancreatic cancer, gallbladder cancer, liver cancer, colorectal cancer, gastric cancer, esophageal cancer, brain glioma, ovarian cancer, cervical cancer, prostate cancer, renal cancer, lung cancer, breast cancer, multiple myeloma, lymphoma, and melanoma.

The key technical points and advantages of the invention are as follows:
1. A synonymously mutated human 4-1BBL gene sequence, which can be used to detect and monitor the expression and distribution of therapeutic (exogenous) 4-1BBL after the recombinant vaccinia virus VV-mH4-1BBL is used to treat tumor patients; and
2. The recombinant vaccinia virus VV-mH4-1BBL based on the synonymously mutated human 4-1BBL gene sequence retains the original oncolytic effect of the oncolytic virus, the characteristics and advantages of initiating anti-tumor immune response and enhancing immune response as an adjuvant, and is safer by deleting TK gene; the 4-1BBL is highly expressed on the surface of tumor cells in a tumor microenvironment by the recombinant vaccinia virus VV-mH4-1BBL, such that the 4-1BBL can enhance anti-tumor immunity by means of stimulating a 4-1BB signal of 4-1BB + immune cells (including T cells) in the tumor microenvironment, and 4-1BBL is also confined within tumor tissues to exert the function thereof in a centralized manner, thereby avoiding the potential systemic toxic side effects caused by the systemic use of the agonistic 4-1BB monoclonal antibody or the soluble 4-1BBL to treat tumors.

Compared with other oncolytic viruses, vaccinia virus has its own characteristics:
1. Mass vaccination has been used to prevent smallpox in all mankind, and its safety has been confirmed; and
2. The weak expression/non-expression of TK gene in normal cells and the strong expression of TK gene in many tumor cells determine that the VV with TK gene knockout is almost not expressed in normal cells, which indicates that the product reconstructed with VV as the parent virus has extremely high safety.

The invention constructs a synonymously mutated gene of human 4-1BBL (the synonymous mutation is that mutation of a certain base pair in a DNA fragment does not change the coded amino acid), and constructs the recombinant oncolytic vaccinia virus VV-mH4-1BBL of synonymously mutated gene of human 4-1BBL on this basis, which retains the oncolytic effect of the original oncolytic virus, the characteristics and advantages of initiating anti-tumor immune response and enhancing immune response as an adjuvant, and is safer by deleting TK gene; the 4-1BBL is highly expressed on the surface of tumor cells in a tumor microenvironment by the recombinant vaccinia virus VV-mH4-1BBL, such that the 4-1BBL can enhance anti-tumor immunity by means of stimulating a 4-1BB signal of 4-1BB + immune cells (including T cells) in the tumor microenvironment, and 4-1BBL is also confined within tumor tissues to exert the function thereof in a centralized manner, thereby avoiding the potential systemic toxic side effects caused by the systemic use of the agonistic 4-1BB monoclonal antibody or the soluble 4-1BBL to treat tumors.

The use of mH4-1BBL in the preparation of a drug for treating a tumor has the following trends and effects:
1. Use of mH4-1BBL in improving anti-tumor immunity of tumor microenvironment:
   The interaction between tumor cells and anti-tumor immunity determines the occurrence and progression of a tumor. The number and functional status of immune cells, especially dendritic cells (DCs) and T cells, in the tumor microenvironment is one of the key factors affecting the efficacy of tumor immunotherapy and the outcome of patients.
   MH4-1BBL dissolves local tumor tissue mainly through the characteristics of oncolytic virus, initiates immune response, and establishes an inflammatory environment of tumor immunity in the local part of the tumor. The mH4-1BBL encoding protein is expressed on the surface of tumor cells to enhance and maintain the anti-tumor functional status of immune cells such as DC and T cells in "the inflammatory environment" of tumor tissues. Therefore, VV-mH4-1BBL has effects on tumor tissues in different immune states: from the perspective of immunophenotyping of tumor microenvironment, VV-mH4-1BBL can be used in "immunoinflammatory type" of tumor tissues (i.e. "a hot tumor", which have been infiltrated by T cells and other immune cells) to make "a hot tumor hotter", that is, to further enhance the number and vitality of immune cells in the tumor microenvironment. VV-mH4-1BBL can be used in "immune desert type" of tumor tissues (i.e. "a cold tumor", which lacks T cells and other immune cells inside and around tumor tissues) to convert "a cold tumor" into "a hot tumor", that is, to promote T cells and other immune cells to infiltrate into tumor tissues, while enhancing their anti-tumor activity and also creating conditions in combination with PD-1 monoclonal antibodies and other immunotherapies. VV-mH4-1BBL can be used in "immune privilege type" of tumor tissues (immune cells such as T cells exist in the periphery of tumor tissues, but cannot infiltrate into tumor tissues), which not only promotes immune cells to break through various barriers and enter tumor tissues, but also promotes the anti-tumor function of immune cells.
2. VV-mH4-1BBL is used to treat superficial and deep tumors by direct injection and combining with endoscopic injection:
   Based on the mode of application (administration), VV-mH4-1BBL can be used for a series of solid tumors existing on the body surface, such as melanoma, by direct intratumoral injection; VV-mH4-1BBL can also be precisely injected into deep solid tumors by using a laparoscopy, a choledochoscopy, a thoracoscopy, an enteroscopy, a neuroendoscopy, etc. to observe tumors clearly, so as to directly dissolve tumor cells and stimulate anti-tumor immune response.
3. VV-mH4-1BBL is used in a drug for the treatment of solid tumors such as colorectal cancer, pancreatic cancer and melanoma:
   In terms of the tumor type, as the mechanism of action of the VV-mH4-1BBL is to directly perform oncolysis and to initiate and enhance the efficiency of immune cells such as T cells, VV-mH4-1BBL can be used for the treatment of pancreatic cancer, gallbladder cancer, liver cancer, colorectal cancer, gastric cancer, esophageal cancer, brain glioma, ovarian cancer, cervical cancer, prostate cancer, renal cancer, lung cancer, breast cancer, multiple myeloma, lymphoma, melanoma, etc. in various clinical stages through the above administration modes.

In fact, VV-mH4-1BBL can be used to treat all types of solid tumors, and the Applicant has also carried out a large number of related experiments to prove this, but considering the limitation of space, only the representative related verification experiments of colorectal cancer, pancreatic cancer and melanoma are listed in this application. This experimental data does not limit the extent to which VV-mH4-1BBL can act on all types of solid tumors.

The invention constructs a synonymously mutated gene mH4-1BBL and further constructs a recombinant vaccinia virus VV-mH4-1BBL. In order to express 4-1BBL molecules with the function of stimulating strong anti-tumor immunity on the surface of tumor cells at a high level as well as detect and monitor the expression of the therapeutic 4-1BBL, a synonymously mutated primer is designed and synthesized molecularly, 540T base is mutated to 540C base through PCR, so as to a synonymously mutated gene mH4-1BBL of 4-1BBL. Further, the mH4-1BBL is recombined into a vaccinia virus genome to construct a novel recombinant vaccinia virus containing a mH4-1BBL gene sequence, which is named as VV-mH4-1BBL. VV-mH4-1BBL not only retains oncolytic properties, but also expresses a large number of proteins encoded by mH4-1BBL gene while replicating rapidly, and expresses on the surface of tumor cells. The recombinant vaccinia virus VV-mH4-1BBL, as a virus itself, can play a role of oncolysis directly and further initiating anti-tumor immunity, and the protein 4-1BBL encoded by the mH4-1BBL gene carried by the same is confined within tumor tissues to exert the function of enhancing the anti-tumor immunity in a centralized manner.

If an agonistic anti-human 4-1BB monoclonal antibody or a soluble 4-1BBL-acting 4-1BB signaling agonist is used for tumor immunotherapy, there may be a potential risk of systemic toxic side effects. The protein 4-1BBL molecule coded by the mH4-1BBL gene carried by the VV-mH4-1BBL is confined within a tumor microenvironment to play the function thereof, so that the potential 4-1BBL systemic side effects can be avoided, and the local 4-1BBL of tumor tissues can be expressed at a high level, so as to play a stronger local anti-tumor effect. This is the advantage of recombinant vaccinia virus VV-mH4-1BBL. In addition, the vaccinia virus thymidine kinase (TK) gene was deleted during the construction of the recombinant virus, which made it safer and further enhanced the selectivity of infecting tumor cells.

The invention well realizes the technical effects of constructing the synonymously mutated gene and the recombinant oncolytic virus, retaining the advantages of the original oncolytic virus and avoiding the disadvantages thereof. In the invention, a 540T base of a wild human 4-1BBL coding sequence is mutated to a 540C base to construct a synonymously mutated gene mH4-1BBL. Then mH4-1BBL was introduced into vaccinia virus VV by homologous recombination to construct recombinant vaccinia virus VV-mH4-1BBL. The recombinant vaccinia virus VV-mH4-1BBL is used in a drug for tumor immunotherapy, 4-1BBL coding fusion protein expressed on the surface of a cell is expressed while the tumor cells are infected and the oncolytic effect is obtained and the local inflammatory environment of the tumor is promoted. While promoting anti-tumor immune response, it avoids potential systemic toxic side effects and other adverse reactions, and breaks through the bottleneck of tumor immunotherapy that anti-tumor immune cells are not easy to enter tumor tissues and anti-tumor immunity is easy to be suppressed by negative immunity in patients.

In the invention, the 4-1BBL is recombined into the oncolytic virus, so that the 4-1BBL can play a role locally in a tumor microenvironment, and the systemic effect is avoided. Therefore, the invention better avoids the potential danger that the super-strong activated 4-1BB signal may be seriously out of control caused by an artificially prepared antibody, and also avoids the potential systemic toxic side effects caused by the direct use of the soluble 4-1BBL.

The synonymously mutated 4-1BBL recombinant vaccinia virus constructed by the invention can monitor the expression of the exogenous 4-1BBL and monitor and determine the curative effect by molecular biology means such as gene sequencing and the like without changing the protein sequence of the 4-1BBL.

### Brief Description of the Drawings

Fig. 1 shows a schematic of a gene fragment introduced into a recombinant vector PV-1 and the PV-1 vector.
Fig. 2 shows a schematic of preparation of a F1-2 fragment during site-directed mutagenesis of human 4-1BBL.
Fig. 3 shows a schematic of preparation of a F1-1 fragment during site-directed mutagenesis of human 4-1BBL.
Fig. 4 shows a schematic of linking site-directed mutagenesis fragments to construct the synonymously mutated full-length human 4-1BBL (mH4-1BBL) gene.
Fig. 5 shows a gel electrophoresis image of a PCR product in which a synonymous mutation is introduced into inside of the human 4-1BBL gene, wherein, Fig. 5A is a gel electrophoresis image of a PCR product of a fragment F1-1 to a fragment F3-2, Fig. 5B shows gel electrophoresis results of PCR products of synonymously mutated mH4-1BBL, mH4-1BBL-2 and mH4-1BBL-3.
Fig. 6 shows the expression of the cell surface 4-1BBL molecule after infection of intestinal cancer cells HCT-116 with VV-ΔTK-4-1BBL, VV-mH4-1BBL, VV-mH4-1BBL-2, and VV-mH4-1BBL-3 as detected by flow cytometry.
Fig. 7 shows the gene sequence of human 4-1BBL expressed in human intestinal cancer cell lines as detected by RT-PCR and gene sequencing.
Fig. 8 shows the alignment of human 4-1BBL gene sequence expressed by human intestinal cancer cell lines by BLAST program of NCBI website.
Fig. 9 shows the gene sequence of human 4-1BBL expressed after VV-mH4-1BBL infection of human intestinal cancer cell lines as detected by RT-PCR and gene sequencing.
Fig. 10 shows the gene sequence of human 4-1BBL expressed after VV-mH4-1BBL-2 infection of human intestinal cancer cell lines as detected by RT-PCR and gene sequencing.
Fig. 11 shows the gene sequence of human 4-1BBL expressed after VV-mH4-1BBL-3 infection of human intestinal cancer cell lines as detected by RT-PCR and gene sequencing.
Fig. 12 shows a schematic of a gene fragment introduced into the recombinant vector PV-1 and the PV-1 vector.
Fig. 13 shows the identification of mouse 4-1BBL gene sequence carried by the PV-4-1BBL recombinant vector by using gene sequencing.
Fig. 14 shows the alignment results of mouse 4-1BBL gene sequence carried by the PV-4-1BBL recombinant vector.
Fig. 15 shows high expression of that 4-1BBL gene after infection of MC-38 cells with the recombinant oncolytic vaccinia virus VVΔTK-4-1BBL as detected by RT-PCR.
Fig. 16 shows that expression of the 4-1BBL molecule on the surface of cells after infection of MC-38 cells with the recombinant oncolytic vaccinia virus VV-ΔTK-4-1BBL as detected by immunofluorescence labeling and flow cytometry.
Fig. 17 shows the ability of VV-ΔTK-4-1BBL to proliferate in different tumor cells as detected by viral titer assay; wherein, A: mouse pancreatic cancer cell line LTPA; B: mouse melanoma cell line B16-F10; C: mouse pancreatic cancer cell line Panc02/Luc; D: mouse intestinal cancer cell line MC-38.
Fig. 18 shows the killing ability of VVΔTK-4-1BBL against different tumor cells in vitro as detected by MTT assay; wherein, A: mouse pancreatic cancer cell line LTPA; B: mouse melanoma cell line B16-F10; C: mouse pancreatic cancer cell line Panc02/Luc; D: mouse intestinal cancer cell line MC-38.
Fig. 19 shows mouse 4-1BBL gene sequence expressed by mouse intestinal cancer cells as detected by gene sequencing.
Fig. 20 shows the alignment results of mouse 4-1BBL gene sequence obtained by RT-PCR.
Fig. 21 shows the gene sequence of 4-1BBL expressed by mouse intestinal cancer cells in VV-ΔTK-4-1BBL-treated group as detected by gene sequencing.
Fig. 22 shows the alignment results of mouse 4-1BBL gene sequence obtained by RT-PCR.
Fig. 23 shows the gross observation of the spleen of MC-38 tumor-bearing mice after VV-ΔTK-4-1BBL treatment and the comparison of the spleen weight and the number of cells in the spleen; wherein, Fig. 23A shows the results of the comparison of the spleen weights, and Fig. 23B shows the results of the comparison of the numbers of cells in the spleen.
Fig. 24 shows the percentages of CD3+T, CD8+T, and CD4+T cells in the spleen after VV-ΔTK-4-1BBL treatment; wherein, Fig. 24A shows the percentage change of CD3+T cells in the spleen, Fig. 24B shows the percentage change of CD8+T cells in the spleen, and Fig. 24C shows the percentage change of CD4+T cells in the spleen.
Fig. 25 shows the effect of VV-ΔTK-4-1BBL treatment on PD-1 expression in CD4+T cells and CD8+T cells in spleen tissue, wherein, Fig. 25A shows the results of immunofluorescence staining and flow cytometry analysis after VV-ΔTK-4-1BBL treatment. Fig. 25B shows a comparison of PD-1 expression in CD8+T cells in the spleen as detected by flow cytometry after VV-ΔTK-4-1BBL treatment, and Fig. 25C shows a comparison of PD-1 expression in CD4+T cells in the spleen as detected by flow cytometry after VV-ΔTK-4-1BBL treatment.
Fig. 26 shows the percentage of CD3+T cells, CD8+T cells, and CD4+T cells in tumor tissues after VV-ΔTK-4-1BBL treatment, wherein, Fig. 26A shows the percentage of CD3+T cells, Fig. 26B shows the percentage of CD8+T cells, and Fig. 26C shows the percentage of CD4+T cells.
Fig. 27 shows changes of immune molecules in tumor tissues after VV-ΔTK-4-1BBL treatment, wherein, Fig. 27A shows changes of immune molecule Perforin, Fig. 27B shows changes of immune molecule Granzyme B, Fig. 27C shows changes of immune molecule IFN-y, Fig. 27D shows changes of immune molecule IL-1β, Fig. 27E shows the change of the immune molecule TNF-α, and Fig. 27F shows the change of the immune molecule TGF-β.
Fig. 28 shows a comparison of therapeutic effects of recombinant vaccinia viruses on an abdominal tumor model of intestinal cancer in mice.
Fig. 29 shows the observation of the survival time of the recombinant oncolytic vaccinia virus VV-ΔTK-4-1BBL in combination with PD-1/PD-L1 blockade for the treatment of the MC-38 abdominal type mouse tumor model.

### Detailed Description of the Invention

In order to make the purpose, technical solution and advantages of the invention more clear, the invention will be further described in detail below. However, it is to be understood that the description herein is merely illustrative of the invention and is not intended to limit the scope of the invention.

Unless otherwise defined, all technical and scientific terms used herein have the same meaning as commonly understood by a person skilled in the art to which the invention belongs, and the terminology used in the description of the invention is for the purpose of describing the detailed embodiments only and is not intended to limit the invention. The reagents and instruments used herein are commercially available, and the characterization means involved can be referred to the relevant description in the prior art, which will not be repeated here.

In order to further understand the present invention, the following detailed description will be made with reference to the preferred embodiments.

A recombinant vaccinia virus of a synonymously mutated gene 4-1BBL (mH4-1BBL), code name VV-mH4-1BBL, is obtained by inserting a synonymously mutated gene mH4-1BBL and a gene sequence of a related regulatory element after the 81001 bp position of maternal genome of a vaccinia virus (the gene sequence is based on GenBank: AY243312.1), wherein the gene sequence inserted into maternal genome of the vaccinia virus is shown in SEQ ID No.22.

Wherein the synonymously mutated gene mH4-1BBL is obtained by selecting the base at position 540 of its coding sequence based on the wild-type human 4-1BBL gene sequence (the gene sequence is based on the NCBI Reference Sequence: NM_003811.4 coding sequence) through designing primers and carrying out synonymous mutation by PCR.

The gene information of the VV-mH4-1BBL recombinant virus is as follows:
1. The gene sequence of the parent vaccinia virus used in construction of the VV-mH4-1BBL recombinant oncolytic virus is based on GenBank: AY243312.1, and the gene sequence of the parent vaccinia virus mentioned in the following text is based on this.
2. Homologous gene recombination is carried out on the synonymously mutated gene mH4-1BBL and related regulatory elements with the parent vaccinia virus by a recombinant vector (PV-mH4-1BBL). In this process, the mH4-1BBL gene and related regulatory elements were inserted after the 81001bp position of the genome of the parent vaccinia virus, that is, in the middle of the thymidine kinase (TK) gene, which not only destroys the complete TK gene (TK gene knockout), but also realizes the expression of mH4-1BBL. Since yellow fluorescent protein (YFP) gene (flanked by loxp sequences) used for recombinant virus screening was included in the related regulatory elements, the mH4-1BBL recombinant vaccinia virus of the invention, VV-mH4-1BBL, was obtained by infecting 293T cells transfected with Cre gene with the above recombinant virus and deleting YFP. Compared with the genome of the parent vaccinia virus (VV), the VV-mH4-1BBL genome has a gene fragment (SEQ ID No.22) inserted into the TK gene, which has the following gene sequence (the underlined sequence is the mH4-1BBL gene; 3' to 5' direction; other sequences are regulatory elements such as the promoter):

SEQ ID No.22:
5'-agatcgataaaaattaattaattacccgggtaccacatttgtagaggttttacttgctttaaaaaacctcccacacctccccctgaacct
gaaacataaaatgaatgcaattgttgttgttaacTTATTCCGACCTCGGTGAAGGAGTCCGGCTGGATTTCG
GGGGTCACCCGGAAGAGTCCCAAGACTGTGGCGCCCTGGGTAAGCTGCCAGGCATGGCGT
GCCCTGGCCTCAGTGTGAAGATGGACGCCCAGGCGCTGGCCGGCACTCAGGTGCAGCAAG
CGGCCCTGGAAACCGAAGGCCGAGTTCCGAGCCTCGGAGGAGGCGGGTGGCAGGTCCAC
GGTCAAAGCCAGGGCGGCGGCCCCAGCAGCAGAGCGCAGTGGCTGCAGGTGCAGCGCAA
GTGAAACGGAGCCTGAGCCCTCGCCGGCCACCACGCGCCGCAGCTCTAGTTGAAAGAAGA
CATAGTAGACTCCAGCCTTGGCCACCACCAGCTCCTTCGTGTCCTCTTTGTAGCTCAGGCCC
CCCGTCAGGGACACGCCTGCCAGGCCTGGGTCACTGTACCAGCTCAGGGGCCCATCGATC
AGCAGAACATTTTGGGCCACCAGCTGCGCAAACATGCCCTGCCGCAGGTCCAAGAGGCCG
GCGGGATCGTCGGGCGAAAGCTCGGGACCCTCGCGGAGTCTCGGGCTGGCCGCGGAGCC
GGGCGAGGCGCGAGCCCCGGACACGGCCCAGGGGCAGGCGAGGAAGACGGCGCAGGCG
GCAGCGAGCAGCAGCAGCAGCAGCAGCCCCGCGACCAGGGCCCAAGGCAGTACGCGGCA
GGCGCGAGCGCGGGGCGCGGAGGCCACGGGGCTTCGGGGTCCAGTGAAGCGTCAGAG
GCGTATTCCATCCTGCAGGGCCGGCCATTATCATCGTGTTTTTCAAAGGAAAACCACGT
CCCCGTGGTTCGGGGGGCCTAGACGTTTTTTAACCTCGACTAAACACATGTAAAGCAT
GTGCACCGAGGCCCCAGATCAGATCCCATACAATGGGGTACCTTCTGGGCATCCTTCA
GCCCCTTGTTGAATACGCTTGAGGTGAGCCATTTGACTCTTTCCACAACTATCCAACTC
ACAACGTGGCACTGGGGTTGTGCCGCCTTTGCAGGTGTATCTTATACACGTGGCTTTTG
GCCGCAGAGGCACCTGTCGCCAGGTGGGGGGTTCCGCTGCCTGCAAAGGGTCGCTAC
AGACGTTGTTTGTCTTCAAGAAGCTTCCAGAGGAACTGCTTCCTTCACGACATTCAAC
AGACCTTGCATTCCTTTGGCGAGAGGGGAAAGACCCCTAGGAATGCTCGTCAAGAAG
ACAGGGCCAGGTTTCCGGGCCCTCACATTGCCAAAAGACGGCAATATGGTGGAAAAT
AACATATAGACAAACGCACACCGGCCTTATTCCAAGCGGCTTCGGCCAGTAACGTTAG
GGGGGGGGGAGGGAGAGGGGCGGCGCGCCTTAAGTCGACttcgagcttatttatattccaaaaaaaa
aaataaaatttcaatttttaagctttcactaattccaaacccacccgctttttatagtaagtttttcacccataaataataaatacaataattaatttctcgt
aaaagtagaaaatatattctaatttattgcacggtaaggaagtagatcataactcgagataacttcgtataatgtatgctatacgaagttatgcggcc
gcttcctcgctcactgacgctagcgccctatagtgagtcgtattacagatcc-3'.

The VV-mH4-1BBL of the invention is established by the following method:
1) Construction of the synonymously mutated gene mH4-1BBL: Taking the wild-type human 4-1BBL gene (the gene sequence is based on the coding sequence of the NCBI Reference Sequence: NM_003811.4), using the base at position 540 of its coding sequence as a center to design and synthesize a synonymously mutated primer, and then 540T base is mutated to 540C base through PCR, only changing the coded polypeptide product, and the synonymously mutated gene 4-1BBL gene is named as a mH4-1BBL gene;
2) Introducing restriction sites into both flanks of the mH4-1BBL gene: introducing Sdal and HpaI restriction sites into both flanks of the mH4-1BBL gene through PCR to separate gene fragments;
3) Preparation of a cohesive end of the vaccinia virus VV homologous recombination vector: the VV homologous recombination vector PV-1 (containing a yellow fluorescent YFP sequence) was digested by using SdaI and HpaI to separate a plasmid fragment;
4) Linking the gene mH4-1BBL and the cohesive end of the VV homologous recombination vector PV-1: linking the recovered products in the step 2) and the step 3);
5) Transforming competent cells and screening positive clones: co-incubating the linked product in the step 4) and competent Escherichia coli, expanding the culture, extracting recombinant plasmids, confirming that the gene mH4-1BBL is correctly cloned into the homologous recombinant vector PV-1 through gene sequencing, named PV-mH4-1BBL;
6) Recombining the gene mH4-1BBL into the VV genome: the PV-mH4-1BBL and the VV are subjected to homologous recombination while the VV is infected and replicated; the mH4-1BBL gene fragment and the yellow fluorescent YFP sequence are simultaneously subjected to homologous recombination into a VV genome to form a recombinant virus; meanwhile, as the position of the mH4-1BBL inserted into the VV genome is inside the TK gene, the TK gene is damaged (i.e., the TK gene is deleted), and then the monoclonal recombinant VV is screened to obtain a high-purity recombinant VV; and
7) Infecting 293T cells expressing cre enzyme with the obtained high-purity recombinant VV, digesting the YFP gene in the recombinant VV genome, and then screening the monoclonal recombinant VV to obtain the seed of the target recombinant VV and purify the same. The correct mH4-1BBL gene was confirmed to be recombined into VV by gene sequencing analysis, and the recombinant oncolytic vaccinia virus of a synonymously mutated gene mH4-1BBL was obtained, named VV-mH4-1BBL.

The invention provides use of a synonymously mutated 4-1BBL recombinant vaccinia virus, namely VV-mH4-1BBL, in the preparation of an antitumor drug. The recombinant vaccinia virus VV-mH4-1BBL can be used alone in the preparation of an anti-tumor drug or used in combination with other anti-tumor drugs or means.

Wherein, the recombinant vaccinia virus VV-mH4-1BBL can be used in the preparation of a drug or a product for treating "immunoinflammatory type" tumor tissues, "immune desert type" tumor tissues and "immune privilege type" tumor tissues.

Wherein, the recombinant vaccinia virus VV-mH4-1BBL can be used in the preparation of a drug or a product for treating superficial and deep tumors by direct injection and combining with endoscopic injection;
Superficial tumors include, but are not limited to, solid tumors such as melanomas that are present on the body surface.

Endoscopic injection therapy includes, but is not limited to, precise intratumoral injection therapy of deep solid tumors based on clear observation of tumors by a laparoscopy, a choledochoscopy, a thoracoscopy, an enteroscopy, a neuroendoscopy, etc.

Wherein, the recombinant vaccinia virus VV-mH4-1BBL can be used in the preparation of a drug or a product for treating a tumor in various clinical stages, wherein the tumor includes, but is not limited to, pancreatic cancer, gallbladder cancer, liver cancer, colorectal cancer, gastric cancer, esophageal cancer, brain glioma, ovarian cancer, cervical cancer, prostatic cancer, renal cancer, lung cancer, breast cancer, multiple myeloma, lymphoma, and melanoma.

The use of the synonymously mutated gene mH4-1BBL is that the gene sequencing of a series of tumors such as intestinal cancer cells and the like shows that the tumor cells themselves express the wild-type 4-1BBL, but its expression product 4-1BBL molecule is located in the cells and is not expressed on the surface of the cells. In the process of treating and intervening tumor cells by using the synonymously mutated gene mH4-1BBL, the gene level detection and the detection of the expression level of the synonymously mutated gene mH4-1BBL can be used for monitoring the treatment process and the curative effect.

### Example 1

Construction of Recombinant Oncolytic Vaccinia Virus: the wild-type human 4-1BBL gene (gene sequence is based on the coding sequence of NCBI Reference Sequence: NM_003811.4) mutated to synonymous codons by PCR site-directed mutagenesis (synonymous mutation refers to the change of nucleotide sequence of gene codons, and the coded amino acid is not changed), the synonymously mutated human 4-1BBL is named as mh4-1BBL, the mh4-1BBL is recombined into a vaccinia virus genome with the TK (thymidine kinase) gene knockout to construct a novel recombinant vaccinia virus, which is named as VV-mh4-1BBL. VV-mh4-1BBL retained the oncolytic properties of the parent VV, and expressed mh4-1BBL molecule after infection with tumor cells and located on the surface of cell membrane. The amino acid sequence of mh4-1BBL molecule was not different from that of wild-type 4-1BBL molecule, but at the mRNA level, it was possible to distinguish whether the 4-1BBL expressed in tumor tissues after VV-mh4-1BBL treatment was endogenous or exogenous. The mh4-1BBL molecule expressed by the oncolytic virus can avoid the potential risk caused by the systemic effect of the use of the soluble 4-1BBL, and also enable the mh4-1BBL molecule to play a therapeutic role in enhancing the anti-tumor immunity in tumor tissues (tumor microenvironment) in a centralized manner.

A recombinant oncolytic vaccinia virus, which is operably inserted with a synonymously mutated exogenous gene capable of expressing 4-1BBL.

An exogenous gene is insert into TK gene of the vaccinia virus.

The DNA sequences of the exogenous gene are shown as SEQ ID No.1 to SEQ ID No.3.

The applicant has listed three synonymously mutated gene sequences, which do not limit the scope of the invention. In fact, as long as the polypeptide/amino acid/protein sequences finally obtained through encoding are the same (same or similar efficacy), the synonymous mutation at any one or more sites is feasible.

The sequence of SEQ ID No.1 (mutation of 540T to C) is:

atggaatacgcctctgacgcttcactggaccccgaagccccgtggcctcccgcgccccgcgctcgcgcctgccgcgtactgccttgg
gccctggtcgcggggctgctgctgctgctgctcgctgccgcctgcgccgtcttcctcgcctgcccctgggccgtgtccggggctcgcgc
ctcgcccggctccgcggccagcccgagactccgcgagggtcccgagctttcgcccgacgatcccgccggcctcttggacctgcggcaggg
catgtttgcgcagctggtggcccaaaatgttctgctgatcgatgggcccctgagctggtacagtgacccaggcctggcaggcgtgtccctgac
gggggggcctgagctacaaagaggacacgaaggagctggtggtggccaaggctggagtctactatgtcttctttcaactagagctgcggcgcg
tggtggccggcgagggctcaggctccgtttcacttgcgctgcacctgcagccactgcgctctgctgctggggccgccgccctggcCttgacc
gtggacctgccacccgcctcctccgaggctcggaactcggccttcggtttccagggccgcttgctgcacctgagtgccggccagcgcctggg
cgtccatcttcacactgaggccagggcacgccatgcctggcagcttacccagggcgccacagtcttgggactcttccgggtgacccccgaaa
tcccagccggactcccttcaccgaggtcggaataa.

The sequence of SEQ ID No.2 (mutation of 468G to A) is:

atggaatacgcctctgacgcttcactggaccccgaagccccgtggcctcccgcgccccgcgctcgcgcctgccgcgtactgccttgg
gccctggtcgcggggctgctgctgctgctgctcgctgccgcctgcgccgtcttcctcgcctgccctgggccgtgtccggggctcgcgc
ctcgcccggctccgcggccagcccgagactccgcgagggtcccgagctttcgcccgacgatcccgccggcctcttggacctgcggcaggg
catgtttgcgcagctggtggcccaaaatgttctgctgatcgatgggcccctgagctggtacagtgacccaggcctggcaggcgtgtccctgac
gggggggcctgagctacaaagaggacacgaaggagctggtggtggccaaggctggagtctactatgtcttctttcaactagagctgcggcgcg
tggtggccggcgaAggctcaggctccgtttcacttgcgctgcacctgcagccactgcgctctgctgctggggccgccgccctggctttgacc
gtggacctgccacccgcctcctccgaggctcggaactcggccttcggtttccagggccgcttgctgcacctgagtgccggccagcgcctggg
cgtccatcttcacactgaggccagggcacgccatgcctggcagcttacccagggcgccacagtcttgggactcttccgggtgacccccgaaa

tcccagccggactcccttcaccgaggtcggaataa.

The sequence of SEQ ID No.3 (mutation of 597C to T) is:

atggaatacgcctctgacgcttcactggaccccgaagccccgtggcctcccgcgccccgcgctcgcgcctgccgcgtactgccttgg
gccctggtcgcggggctgctgctgctgctgctcgctgccgcctgcgccgtcttcctcgcctgcccctgggccgtgtccggggctcgcgc
ctcgcccggctccgcggccagcccgagactccgcgagggtcccgagctttcgcccgacgatcccgccggcctcttggacctgcggcaggg
catgtttgcgcagctggtggcccaaaatgttctgctgatcgatgggcccctgagctggtacagtgacccaggcctggcaggcgtgtccctgac
gggggggcctgagctacaaagaggacacgaaggagctggtggtggccaaggctggagtctactatgtcttctttcaactagagctgcggcgcg
tggtggccggcgagggctcaggctccgtttcacttgcgctgcacctgcagccactgcgctctgctgctggggccgccgccctggctttgaccg
tggacctgccacccgcctcctccgaggctcggaactcggccttcggtttTcagggccgcttgctgcacctgagtgccggccagcgcctggg
cgtccatcttcacactgaggccagggcacgccatgcctggcagcttacccagggcgccacagtcttgggactcttccgggtgacccccgaaa
tcccagccggactcccttcaccgaggtcggaataa.

The amino acid sequence of the exogenous gene is shown as SEQ ID No.4.

The sequence of the SEQ ID No.4 is:

MEYASDASLDPEAPWPPAPRARACRVLPWALVAGLLLLLLLAAACAVFLACPWAVSG
ARASPGSAASPRLREGPELSPDDPAGLLDLRQGMFAQLVAQNVLLIDGPLSWYSDPGLAG
VSLTGGLSYKEDTKELVVAKAGVYYVFFQLELRRVVAGEGSGSVSLALHLQPLRSAAGAA
ALALTVDLPPASSEARNSAFGFQGRLLHLSAGQRLGVHLHTEARARHAWQLTQGATVLGL
FRVTPEIPAGLPSPRSE.

The exogenous gene is linked via an IRES sequence.

IRES is a ribosome entry site and is only one of the linker sequences that can be used.

The IRES sequence is shown as SEQ ID No.5.

The sequence of the SEQ ID No.5 is:

TTATCATCGTGTTTTTCAAAGGAAAACCACGTCCCCGTGGTTCGGGGGGCCTAGAC
GTTTTTAACCTCGACTAAACACATGTAAAGCATGTGCACCGAGGCCCCAGATCAGATC
CCATACAATGGGGTACCTTCTGGGCATCCTTCAGCCCCTTGTTGAATACGCTTGAGGTG
AGCCATTTGACTCTTTCCACAACTATCCAACTCACAACGTGGCACTGGGGTTGTGCCGC
CTTTGCAGGTGTATCTTATACACGTGGCTTTTGGCCGCAGAGGCACCTGTCGCCAGGTG
GGGGGTTCCGCTGCCTGCAAAGGGTCGCTACAGACGTTGTTTGTCTTCAAGAAGCTTC
CAGAGGAACTGCTTCCTTCACGACATTCAACAGACCTTGCATTCCTTTGGCGAGAGGG
GAAAGACCCCTAGGAATGCTCGTCAAGAAGACAGGGCCAGGTTTCCGGGCCCTCACAT
TGCCAAAAGACGGCAATATGGTGGAAAATAACATATAGACAAACGCACACCGGCCTTAT
TCCAAGCGGCTTCGGCCAGTAACGTTAGGGGGGGGGGAGGGAGAGGGG.

The invention also provides a pharmaceutical composition, which comprises the above recombinant oncolytic vaccinia virus, a pharmaceutically acceptable carrier and a pharmaceutical excipient.

The administration mode of the pharmaceutical composition is direct injection and/or injection in combination with an endoscope; and the endoscope is preferably a laparoscope, a choledochoscope, a thoracoscope, an enteroscope, and a neuroendoscope.

The invention also provides use of the above recombinant oncolytic vaccinia virus or the above pharmaceutical composition in the preparation of a drug for preventing or treating a tumor and/or a cancer.

The tumor and/or cancer is a solid tumor, preferably a cold tumor and/or a hot tumor, and the histological types thereof include, but are not limited to, pancreatic cancer, gallbladder cancer, liver cancer, colorectal cancer, gastric cancer, esophageal cancer, brain glioma, ovarian cancer, cervical cancer, prostate cancer, renal cancer, lung cancer, breast cancer, multiple myeloma, lymphoma, and melanoma.

### Example 2

### Detection of synonymous mutation sites of the synonymously mutated human 4-1BBL recombinant vaccinia virus after infecting intestinal cancer cells:

The biological function of a protein molecule depends on its correct amino acid sequence. The base sequence of DNA encodes amino acids through triple codons. In this process, there is a biological phenomenon of "degenerate codon", that is, one amino acid can be encoded by more than one triplet code. Therefore, different codons corresponding to the same amino acid are referred to as synonymous codons. These synonymous codons are usually the same at the first and second bases, and differ only at the third base.

In view of the above biological mechanism, synonymous mutation (changing the third base of the codon and keeping the amino acid encoded by the codon unchanged) can be performed on the codons of the DNA sequence of the gene encoding a specific protein. In this way, not only the amino acid sequence and function of the gene expression product are not affected, but also the synonymous mutation base introduced into the cell can be detected by means of molecular biology.

In this example, the recombinant oncolytic virus expressing human 4-1BBL gene with synonymous mutation was constructed to express 4-1BBL molecule locally in tumor tissues to promote anti-tumor immunity while exerting the anti-tumor effect of oncolytic virus itself, and to detect therapeutic (exogenous) 4-1BBL by detecting synonymous mutation sites.

### I. Construction of the recombinant vector carrying the synonymously mutated human 4-1BBL gene:

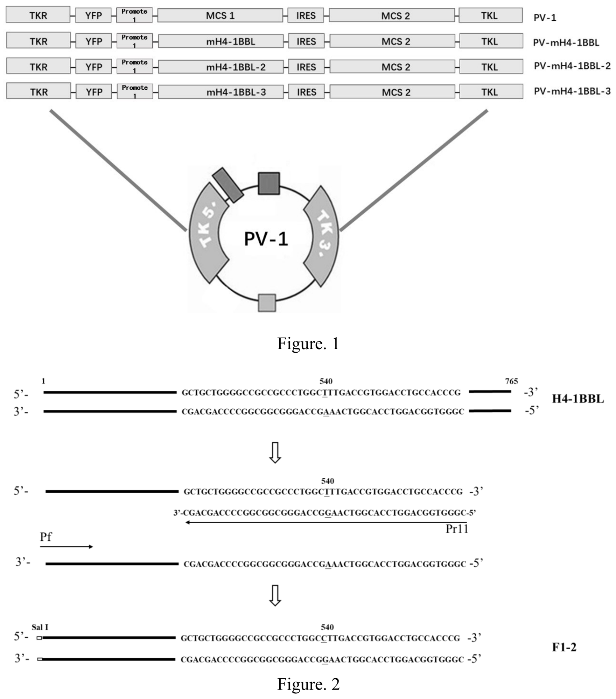

In order to verify that the mutated human 4-1BBL gene expressed after the synonymously mutated human 4-1BBL recombinant vaccinia virus infects tumor cells can be detected by molecular biological means, the synonymously mutated human 4-1BBL gene was constructed in this example and cloned into the homologous recombination vector PV-1 (Fig. 1). The synonymously mutated human 4-1BBL gene was introduced into vaccinia virus by homologous recombination, and the TK (thymidine kinase) gene was deleted at the same time to obtain the recombinant vaccinia virus of synonymously mutated human 4-1BBL.

The gene fragment introduced into the recombinant vector PV-1 and the structure of the PV- 1 vector are schematically shown in Fig. 1. In the vector of Fig. 1, TK 5' and TK 3' sequences are homologous to portions of the vaccinia virus genome, flanking the gene sequence of interest. The recombinant vector was introduced to the host cells of the wild-type vaccinia virus. The TK 5' and TK 3' sequences undergo homologous recombination with the wild-type vaccinia virus genes. The gene sequences between TK 5' and TK 3' in the vector were introduced into the vaccinia virus genome to construct a recombinant virus.

The nouns in Fig. 1 are:
1) MCS: a multiple cloning site into which a target gene can be inserted;
2) YFP: yellow fluorescent protein gene;
3) IRES: ribosome entry site;
4) PV-1: homologous recombination vector;
5) PV-mH4-1BBL: a recombinant vector in which mH4-1BBL gene is introduced into the multiple cloning site of the PV-1 vector;
6) PV-mH4-1BBL-2: a recombinant vector in which mH4-1BBL-2 gene is introduced into the multiple cloning site of the PV-1 vector;
7) PV-mH4-1BBL-3: a recombinant vector in which mH4-1BBL-3 gene is introduced into the multiple cloning site of the PV-1 vector.

The above mH4-1BBL, mH4-1BBL-2, and mH4-1BBL-3 genes are based on the CDS of the wild-type human 4-1BBL gene (NCBI Reference Sequence: NM_003811.4), and when the base T at position 540 has been mutated to the base C, the base G at position 468 has been mutated to the base A, and the base C at position 597 has been mutated to the base T, the sequence of the encoded polypeptide product is the synonymously mutated 4-1BBL gene.

### II. Preparation of the synonymously mutated human 4-1BBL gene:

1. Design and preparation of synonymous mutation of the base T at position 540 in the CDS of human 4-1BBL gene to the base C at position 540:
   In order to construct a synonymously mutated human 4-1BBL gene based on the coding sequence (CDS) of wild-type human 4-1BBL gene (H4-1BBL), on the basis of the CDS of the wild-type human 4-1BBL gene (NCBI Reference Sequence: NM_003811.4), a mutation primer was designed for the base T at position 540 of the CDS and mutated to the base C by PCR. However, in the CDS of human 4-1BBL gene, the codon of the base T at position 540 is "GCT" (encoding alanine), which still encodes alanine after mutation to "GCC". The primers were designed as follows:
   Pf (SEQ ID No.6):
   5'-TATAGTCGACATGGAATACGCCTCTGACGCTTC-3' (with Sal I restriction site);
   Meanwhile, a synonymously mutated primer is designed and synthesized by taking the base at position 540 of the coding sequence as a center:
      Pr11 (SEQ ID No.7):
      5'-CGGGTGGCAGGTCCACGGTCAAGGCCAGGGCGGCGGCCCCAGCAGC-3'.

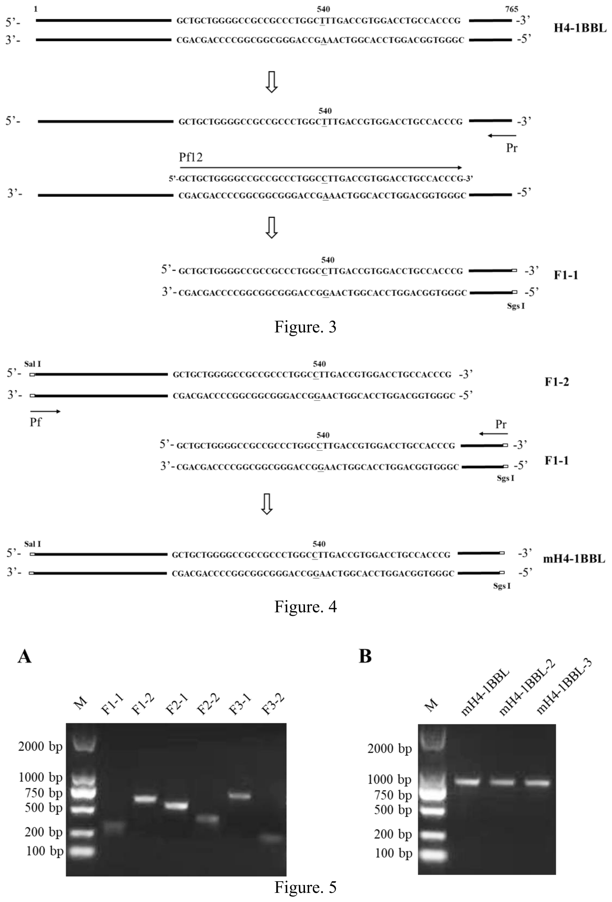

Fragment F1-2 was obtained by PCR using the wild-type human 4-1BBL gene (H4-1BBL) as a template as well as Pf and Pr11 as primers (see Fig. 2 for the technical route and Fig. 5 for the PCR product fragment).

In order to construct a synonymously mutated human 4-1BBL gene based on the wild-type human 4-1BBL gene (H4-1BBL), on the basis of the coding sequence (CDS) of the wild-type human 4-1BBL gene (NCBI Reference Sequence: NM_003811.4), the base T at position 540 of CDS was selected, and primers were designed to mutate to the base C by PCR. The primers were designed as follows:
Pr (SEQ ID No.8):
5'-TATAGGCGCGCCTTATTCCGACCTCGGTGAAGGGAG-3' (with Sgs I restriction site);
Meanwhile, a synonymously mutated primer is designed and synthesized by taking the base at position 540 of the coding sequence as a center:
   Pf12 (SEQ ID No.9):
   5'-GCTGCTGGGGCCGCCGCCCTGGCCTTGACCGTGGACCTGCCACCCG-3'.

Fragment F1-1 was obtained by PCR using the wild-type human 4-1BBL gene (H4-1BBL) as a template as well as Pf12 and Pr as primers (see Fig. 3 for the technical route and Fig. 5 for the PCR product fragment).

In order to link the F1-1 and F1-2 fragments containing the mutated base to a full-length human 4-1BBL gene containing the mutation, the full-length human 4-1BBL gene fragment with the base at position 540 mutated to C was obtained by PCR using the F1-1 and F1-2 fragments as templates as well as Pf and Pr as primers. Compared with the coding sequence (CDS) of the wild-type human 4-1BBL gene, the obtained fragment has a change at the base at position 540 of the CDS. Since the sequence of the encoded polypeptide product is not changed at the third position of the codon to which it belongs, it is called the synonymously mutated 4-1BBL gene and named mH4-1BBL (see Fig. 4 for the technical route and Fig. 5 for the PCR product fragment).

Primer information is as follows:
Pf(SEQ ID No.10):
   5'-TATAGTCGACATGGAATACGCCTCTGACGCTTC-3' (with Sal I restriction site);
Pr (SEQ ID No.11):
   5'-TATAGGCGCGCCTTATTCCGACCTCGGTGAAGGGAG-3' (with Sgs I restriction site).

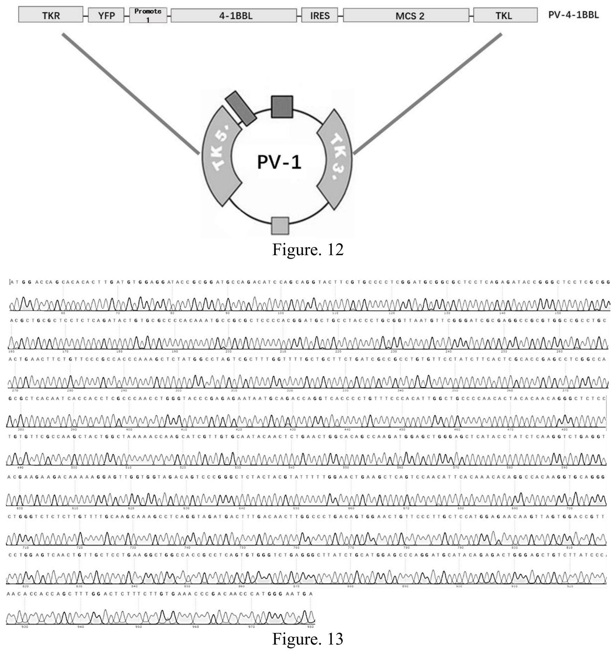

2. Synonymous mutation design and preparation process for the base G at position 468 and the base C at position 597 in the CDS of the human 4-1BBL gene:
In order to demonstrate the universality of the action of the synonymous mutation of the 4-1BBL gene in this example, using procedures similar to those shown in Fig. 2, Fig.3 and Fig.4, the base G at position 468 and the base C at position 597 of the coding sequence of the wild-type human 4-1BBL gene (gene sequence is based on NCBI Reference Sequence: NM_003811.4) were selected for synonymous mutation, respectively. Details are as follows:
1) designing a synonymously mutated primer by taking the base at position 468 of the coding sequence as a center:
   Pr21 (SEQ ID No.12):
      5'- CAAGTGAAACGGAGCCTGAGCCTTCGCCGGCCACCACGCGC-3';
   Pf22 (SEQ ID No.13):
      5'- GCGCGTGGTGGCCGGCGAAGGCTCAGGCTCCGTTTCACTTG-3'.
   The wild-type human 4-1BBL gene was used as a template, Pf and Pr21 were used as a primer pair, Pf22 and Pr were used as a primer pair, and the synonymously mutated base at position 468 (the base G at position 468 is mutated to the base A, and the codon still encodes glutamic acid) was introduced into the human 4-1BBL gene fragment through high-fidelity PCR, and PCR product fragments F2-1 and F2-2 were recovered (Fig. 13). Fragments F2-1 and F2-2 were used as templates, and F and R were used as genes to link the fragments F2-1 and F2-2 to a complete human 4-1BBL gene with a synonymous mutation at the base at position 468. The mutation of the base at position 468 did not change the sequence of the encoded polypeptide product, and the synonymously mutated 4-1BBL gene was named as mH4-1BBL-2 gene (see Fig. 5 for the PCR product fragment).
2) designing a synonymously mutated primer by taking the base at position 597 of the coding sequence as a center:
   Pr31 (SEQ ID No.14):
      5'- GGTGCAGCAAGCGGCCCTGAAAACCGAAGGCCGAGTTCCG-3';
   Pf32 (SEQ ID No.15):
      5'- CGGAACTCGGCCTTCGGTTTTCAGGGCCGCTTGCTGCACC-3'.

The wild-type human 4-1BBL gene was used as a template, Pf and Pr31 were used as a primer pair, Pf32 and Pr were used as a primer pair, and the synonymously mutated base at position 597 (the base C at position 597 is mutated to the base T, and the codon still encodes glutamic acid) was introduced into the human 4-1BBL gene fragment through high-fidelity PCR, and PCR product fragments F3-1 and F3-2 were recovered (Fig. 13). Fragments F3-1 and F3-2 were used as templates, and F and R were used as genes to link the fragments F3-1 and F3-2 to a complete human 4-1BBL gene with a synonymous mutation at the base at position 597. The mutation of the base at position 597 did not change the sequence of the encoded polypeptide product, and the synonymously mutated 4-1BBL gene was named as mH4-1BBL-2 gene (see Fig. 5 for the PCR product fragment).

3. Preparation of homologous recombination vector of synonymously mutated human 4-1BB:
The vector PV-1, mH4-1BBL gene, mH4-1BBL-2 gene and mH4-1BBL-3 gene were digested by Sal I and Sgs I restriction enzymes, respectively. After 1% gel electrophoresis, the gel was digested and the target fragments were recovered. The digested vector PV-1 backbone by T4 ligase was linked to mH4-1BBL gene, mH4-1BBL-2 gene and mH4-1BBL-3 gene, respectively (overnight at 4°C), and the linked products were transformed into DH5α-competent E. coli and spread on LB plates containing 100 µg/mL ampicillin. Culturing at 37°C for 16 hours, selecting resistant colonies, extracting recombinant plasmids, and confirming that the mH4-1BBL gene, the mH4-1BBL-2 gene and the mH4-1BBL-3 gene were correctly cloned into a homologous recombinant vector PV-1 by gene sequencing; the above recombinant plasmids were named as PV-mH4-1BBL, PV-mH4-1BBL-2 and PV-mH4-1BBL-3, respectively.

### III. Preparation of recombinant oncolytic virus of a synonymously mutated human 4-1BBL:

PV-mH4-1BBL, PV-mH4-1BBL-2 and PV-mH4-1BBL-3 were used to infect CV-1 cells with Vaccinia Virus (VV), respectively, and recombinant oncolytic viruses were obtained.

CV1 cells in logarithmic growth phase were taken, the cell density was adjusted to 1 ×10⁵ cells/mL, and inoculated into a 24-well plate according to 1 mL/well. After 24 h, CV1 cells were infected with Vaccinia Virus (VV). Two hours after infection, PV-mH4-1BBL, PV-mH4-1BBL-2, and PV-mH4-1BBL-3 were mixed with transfection reagent (Polyplus, France) and incubated at room temperature for 15 min, and 200 µL of the mixture was taken to transfect CV1 cells. DMEM medium containing 10% FCS was added after 2 h, and the culture was continued for 48 h. The fluorescence intensity of yellow fluorescent protein (YFP) was detected by fluorescence microscopy to determine the homologous recombination between the plasmid and the virus. After the cells were completely broken, the virus cell suspension was collected.

CV1 cells in logarithmic growth phase were taken, the cell density was adjusted to 1.5 ×10⁵ cells/mL, and inoculated into a 6-well plate according to 2 mL/well. After 24 h, CV1 cells were infected by adding the virus cell suspension. After 24 h of culture, CV1 cells were digested, and YFP-expressing CV1 cells were sorted by flow cytometry to prepare a single cell suspension. YFP + CV1 cells were inoculated into a 96-well plate pre-plated with CV1 cells by limiting dilution. After 48 h, a culture well with a single YFP + virus plaque was selected, that cells and the supernatant was collected, and the virus suspension was obtained after repeated freezing and thawing.

The obtained virus suspension was used for infecting 293 T cells expressing the cre enzyme, the YFP gene in the recombinant VV genome was digested, then the monoclonal recombinant VV was screened, and the seed of the target recombinant VV was obtained and purified. The gene sequencing analysis confirms that synonymously mutated human 4-1BBL genes mH4-1BBL, mH4-1BBL-2 and mH4-1BBL-3 were recombined into the VV. The recombinant oncolytic vaccinia virus into which the synonymously mutated human 4-1BBL genes mH4-1BBL, mH4-1BBL-2, and mH4-1BBL-3 were inserted was named as VV-mH4-1BBL, VV-mH4-1BBL-2, and VV-mH4-1BBL-3.

Hela cells were cultured in a 15 cm diameter of culture dish. When the abundance of Hela cells was about 80%, the above virus cell suspension was added to infect Hela cells, and the infection was amplified until enough virus cell suspension was obtained.

Purifying the recombinant oncolytic vaccinia virus: collecting virus cell suspension, centrifuging and discarding supernatant. The cells were lysed by freezing and thawing for three times, and the cells were grounded by Downs homogenizer to release the virus particles. The virus-containing supernatant was collected by centrifugation at 1200 r/min and added to 36% sucrose solution. Centrifugation was performed at 130000 ×g for 1 h. The obtained precipitate was resuspended in 10 mM Tris-HCl and frozen for later use.

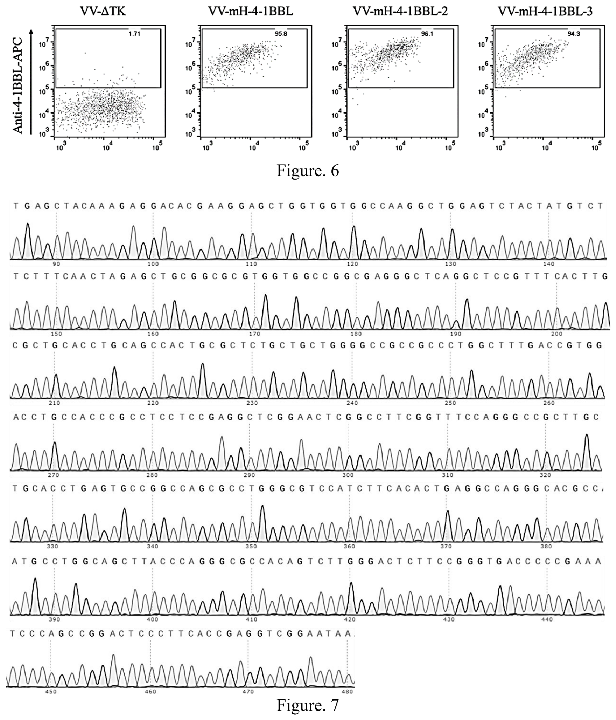

In order to confirm whether the 4-1BBL molecule can be expressed on the surface of tumor cells after the constructed synonymously mutated human 4-1BBL recombinant vaccinia virus infects tumor cells, the expression of the 4-1BBL molecule on the surface of the cells was detected by immunofluorescence labeling and flow cytometry analysis after the virus infects the human intestinal cancer cell lines, as follows:
Human intestinal cancer cell line HCT-116 in logarithmic growth phase was taken, and the cell density was adjusted to 3 ×10⁵ cells/mL, and 1 mL was inoculated into a 24-well plate. When the cells were completely adherent, they were divided into VV-ΔTK, VV-mH4-1BBL, VV-mH4-1BBL-2 and VV-mH4-1BBL-3 infection groups, and infected according to the multiplicity of infection (MOI) of 1. After 24 h, the infected cells were collected and stained with APC-labeled anti-human 4-1BBL monoclonal antibody, and the expression of 4-1BBL on the cell membrane was analyzed by flow cytometry. The results show that the 4-1BBL molecule is not expressed on the surface of the HCT-116 cell infected with the vaccinia virus VV-ΔTK with the tk gene deleted only, and HCT-116 cells infected with VV-mH4-1BBL, VV-mH4-1BBL-2, and VV-mH4-1BBL-3 expressed 4-1BBL molecule at high levels on the cell surface (Fig. 6).

In Fig. 6, VV-ΔTK: a vaccinia virus in which the tk gene is deleted; VV-mH4-1BBL: a recombinant vaccinia virus in which the tk gene is deleted and the mH4-1BBL gene fragment is inserted; VV-mH4-1BBL-2: a recombinant vaccinia virus in which the tk gene is deleted and the mH4-1BBL-2 gene fragment is inserted; VV-mH4-1BBL-3: recombinant vaccinia virus with tk gene deleted and mH4-1BBL-3 gene inserted; Anti-4-1BBL-APC: APC-labeled anti-human 4-1BBL monoclonal antibody.

### IV. After the tumor cells were infected by the synonymously mutated human 4-1BBL recombinant oncolytic vaccinia virus, the synonymous mutation sites were detected by a molecular biology means:

In order to verify whether the introduced exogenous 4-1BBL can be detected after the recombinant oncolytic vaccinia virus of synonymously mutated human 4-1BBL infects the tumor cells, the recombinant oncolytic viruses VV-mH4-1BBL, VV-mH4-1BBL-2 and VV-mH4-1BBL-3 were used to infect the human intestinal cancer cell line HCT-116, respectively, and the uninfected group was set as the control group. The analysis was then detected by RT-PCR as follows:
Human intestinal cancer cell line HCT-116 was infected with VV-mH4-1BBL, VV-mH4-1BBL-2 and VV-mH4-1BBL-3 according to MOI = 1 as described above, and the uninfected group was set. After 24 h, the cells were collected and lysed in 200 µL Trizol solution, and the total RNA was extracted. The RNA was reversely transcribed into cDNA by Takara's RT-PCR kit. At the same time, total RNA was extracted from uninfected HCT116 cells and cDNA was obtained by reverse transcription.

Primers were designed in the CDS region of the human 4-1BBL gene (gene sequence is based on NCBI Reference Sequence: NM_003811.4) covering the mutation site, and the primer sequences were as follows:
Pif (SEQ ID No.16): 5'-TGAGCTACAAAGAGGACACGAAGGAGC-3';
PiR (SEQ ID No.17): 5'-TTATTCCGACCTCGGTGAAGGGAGTCC-3'.

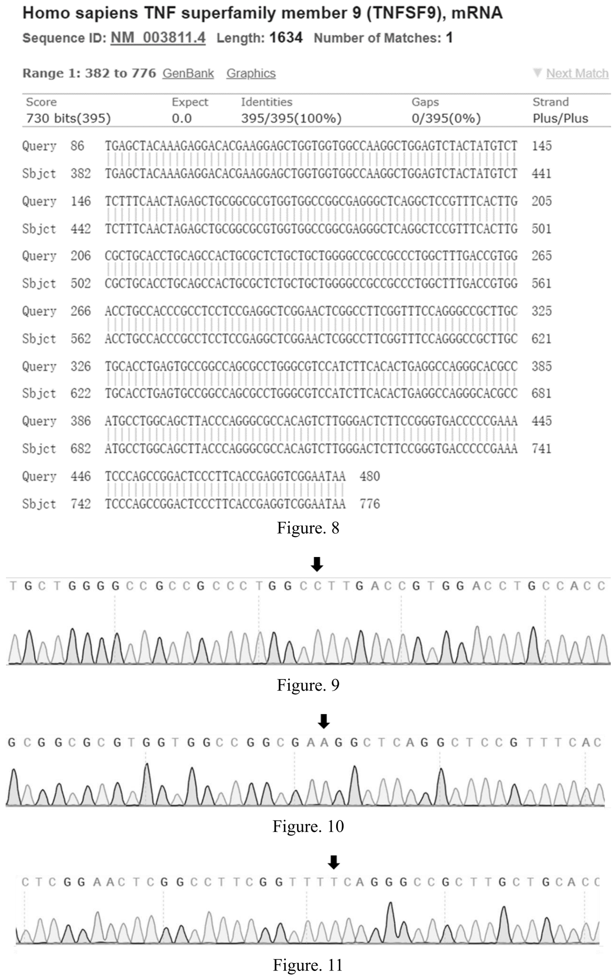

The obtained cDNA was used as a template, Pif and PiR were used as primers, a human 4-1BBL gene fragment covering the synonymous mutation sites was amplified by PCR, a PCR product was linked to a pUMc-T vector, transformed into competent DH5α Escherichia coli, spread on an ampicillin resistant LB plate, after formation of resistant colonies, single colonies were randomly selected for one-way sequencing. The sequencing primer sequence was 5'-GTTGTAAAACG ACGGCCAG-3' (SEQ ID No.18). The results are as follows:
Five colonies in the uninfected group were selected for plasmid extraction and gene sequencing, and the results were shown in Fig. 7, alignment of the BLAST program of NCBI website showed that the fragment covered by the primers Pif and Pir was 100% match with the CDS region of the human 4-1BBL gene (NCBI Reference Sequence: NM_003811.4) (Fig. 8). The results confirmed that the human 4-1BBL gene expressed by the intestinal cancer cells had no mutation.
1) 10 colonies of the VV-mH4-1BBL infection group were selected for one-way sequencing. The results showed that a total of 10 colonies were successfully sequenced, and synonymous mutations as indicated by arrows in Fig. 9 were detected, while no mutations were detected at other sites. The results indicated that VV-mH4-1BBL can replicate in large quantities after infection of tumor cells, and the synonymous mutation (mutation of the base T at position 540 to the base C at position 540) carried by VV-mH4-1BBL can be clearly detected. Therefore, the synonymous mutation of human 4-1BBL can provide a means for detecting and identifying the introduction of exogenous human 4-1BBL gene without changing the encoded amino acid sequence.
2) 10 colonies of the VV-mH4-2 infection group were selected for one-way sequencing. The results showed that a total of 10 colonies were successfully sequenced, and synonymous mutations as indicated by arrows in Fig. 10 were detected, while no mutations were detected at other sites. The results indicated that VV-mH4-1BBL can replicate in large quantities after infection of tumor cells, and the synonymous mutation (mutation of the base G at position 468 to the base A at position 468) carried by VV-mH4-1BBL can be clearly detected. Therefore, the synonymous mutation of human 4-1BBL can provide a means for detecting and identifying the introduction of exogenous human 4-1BBL gene without changing the encoded amino acid sequence.
3) 10 colonies of the VV-mH4-2 infection group were selected for one-way sequencing. The results showed that a total of 10 colonies were successfully sequenced, and synonymous mutations as indicated by arrows in Fig. 11 were detected, while no mutations were detected at other sites. The results indicated that VV-mH4-1BBL can replicate in large quantities after infection of tumor cells, and the synonymous mutation (mutation of the base C at position 597 to the base T at position 597) carried by VV-mH4-1BBL can be clearly detected. Therefore, the synonymous mutation of human 4-1BBL can provide a means for detecting and identifying the introduction of exogenous human 4-1BBL gene without changing the encoded amino acid sequence.

### Example 3

### Antitumor effect of synonymously mutated 4-1BBL recombinant vaccinia virus and detection of synonymous mutation sites:

In order to further verify the anti-tumor effect of the synonymously mutated 4-1BBL recombinant vaccinia virus and verify the detection of the synonymously mutated 4-1BBL in the treatment process, the synonymous mutant based on mouse 4-1BBL was constructed in this example, and the synonymously mutated mouse 4-1BBL oncolytic vaccinia virus was constructed. At the same time, the mouse model of abdominal intestinal cancer was established for experimental treatment and the synonymous mutation sites of mouse 4-1BBL were detected during the treatment. The specific implementation process is as follows:

### I. Construction of a synonymously mutated mouse 4-1BBL gene recombinant vector and a synonymously mutated mouse 4-1BBL recombinant oncolytic vaccinia virus:

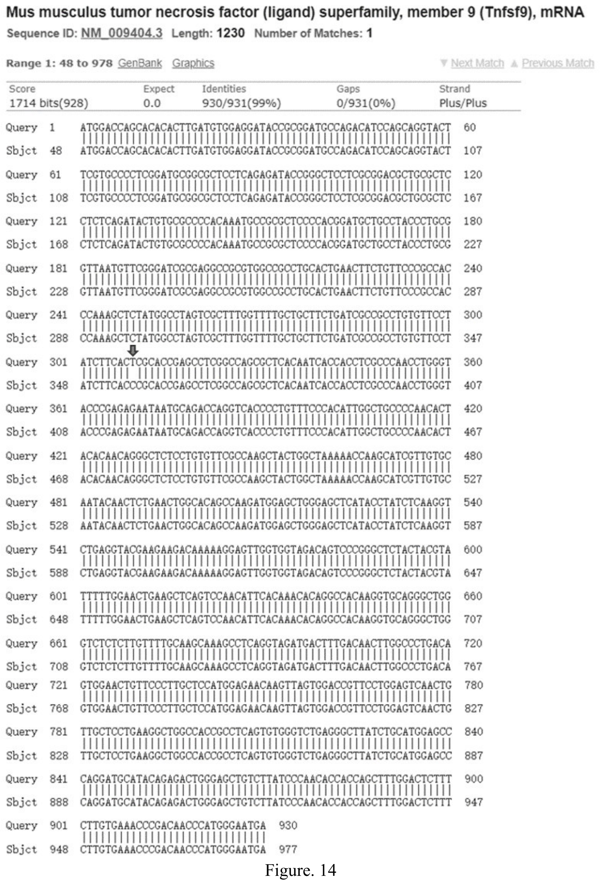

In order to verify that the mutated human 4-1BBL gene expressed after infection of tumor cells with the synonymously mutated human 4-1BBL recombinant vaccinia virus can be detected by molecular biological means, the synonymously mutated mouse 4-1BBL gene was constructed by a similar procedure similar to that in Example 2, and cloned into the homologous recombination vector PV-1 (Fig. 12), named as PV-4-1BBL. The gene sequencing identification results were shown in Fig. 13, and alignment of BLAST program of NCBI website showed that compared with CDS of mouse 4-1BBL gene (NCBI Reference Sequence ID: NM_009404.3), the base C at position 309 of the CDS of the 4-1BBL gene in PV-4-1BBL was mutated to the base T (indicated by the arrow in Fig. 14), that is, the codon ACC to which it belongs was mutated to ACT, but the encoded product of both was still threonine. Thus, a homologous recombinant vector based on the synonymously mutated mouse 4-1BBL gene (referred to as 4-1BBL in this example) was successfully established.

By using a procedure similar to that in example 2, the synonymously mutated 4-1BBL gene was recombined into the oncolytic vaccinia virus by using the PV-4-1BBL, with the tk gene knockout, the recombinant oncolytic virus was established and named as VV-ΔTK-4-1BBL, the anti-tumor function of VV-ΔTK-4-1BBL was verified by in vivo and in vitro experiments, and the synonymous mutation sites of 4-1BBL were detected.

PV-4-1BBL in Fig. 12: recombinant vector after introduction of a synonymously mutated mouse 4-1BBL gene into the multiple cloning site of PV-1 vector.

### II. RT-PCR was used to detect the expression of 4-1BBL in VV-ΔTK-4-1BBL infected intestinal cancer cells at mRNA level:

In order to detect the expression of 4-1BBL gene in tumor cells infected by VV-ΔTK-4-1BBL, MC-38 cells were selected as the model, and the MC-38 cells were infected by VVΔTK-4-1BBL at the multiplicity of infection (MOI) of 1, and the VV-ΔTK infection group was set as the control. Tumor cells were collected 24 h after infection, and total RNA was extracted and reversely transcribed into cDNA, and 4-1BBL gene was amplified by PCR.

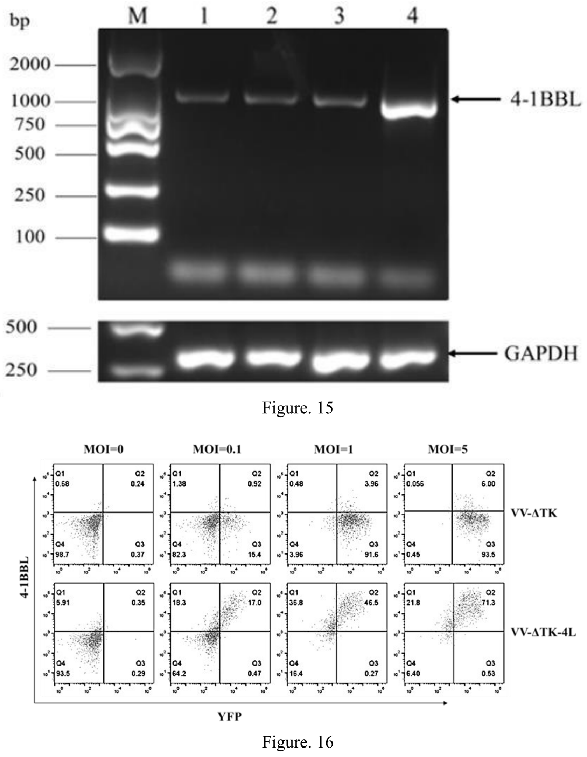

The results of the experiment (Fig. 15) showed that the target gene band in the VV-ΔTK-4L infection group was highlighted, while the VV-ΔTK infection group and the non-infection group had only the target gene band at the background level. This result confirms that the tumor cells infected with VVΔTK-4-1BBL expressed high levels of the 4-1BBL gene by virtue of the rapid replication of the virus, and also demonstrates that there is a background level of 4-1BBL gene expression in the tumor cells (the applicant's previous study found that the protein encoded by this gene was expressed within the tumor cell membrane without intervention).

After the recombinant oncolytic vaccinia virus VV-ΔTK-4-1BBL was used to infect MC-38 cells, RT-PCR was used to detect the high expression of the 4-1BBL gene, as shown in Fig. 15, wherein, Lane M: DL 2000 DNA Marker; Lane 1: uninfected group; Lane 2: VV-ΔTK infected group; Lane 3: uninfected group; Lane 4: VVΔTK-4-1BBL infected group.

### III. The expression of 4-1BBL on the surface of intestinal cancer cells infected by VV-ΔTK-4-1BBL was detected by flow cytometry:

In order to confirm whether the recombinant oncolytic virus could be used for rapid replication in tumor cells to achieve high expression of 4-1BBL molecule and localized on the surface of tumor cells, intestinal cancer cell line MC-38 in logarithmic growth phase was infected according to different virus multiplicity of infection (MOI) of 0, 0.1, 1 and 5, and the cells were digested after 24 h to prepare a single cell suspension, which was stained by a fluorescein-labeled anti-4-1BBL monoclonal antibody, and the expression of 4-1BBL on cell membrane was analyzed by flow cytometry.

The experimental results (Fig. 16) showed that the expression of yellow fluorescent protein (YFP) carried by the recombinant virus increased with the increase of the multiplicity of infection (MOI); the VV-ΔTK-4L infection group expressed membrane-type 4-1BBL, and increased with the increase of MOI; the VV-ΔTK infection group did not express membrane-type 4-1BBL. This result confirmed that 4-1BBL molecule could be expressed on the surface of tumor cells after VV-ΔTK-4-1BBL infection.

### IV. Detection of the proliferation ability of VV-ΔTK-4-1BBL in different tumor cells by virus titer assay:

In order to confirm the effect of gene recombination on the replication and proliferation of vaccinia virus in tumor cells, LTPA cells, B16-F10 cells, Panc02/Luc cells and MC-38 cells were infected with recombinant viruses, and the cells were collected at 24 h, 48 h and 72 h after infection, respectively. After repeated freeze-thawing and dilution, CV-1 cells were infected, and the virus yield was determined by plaque assay.

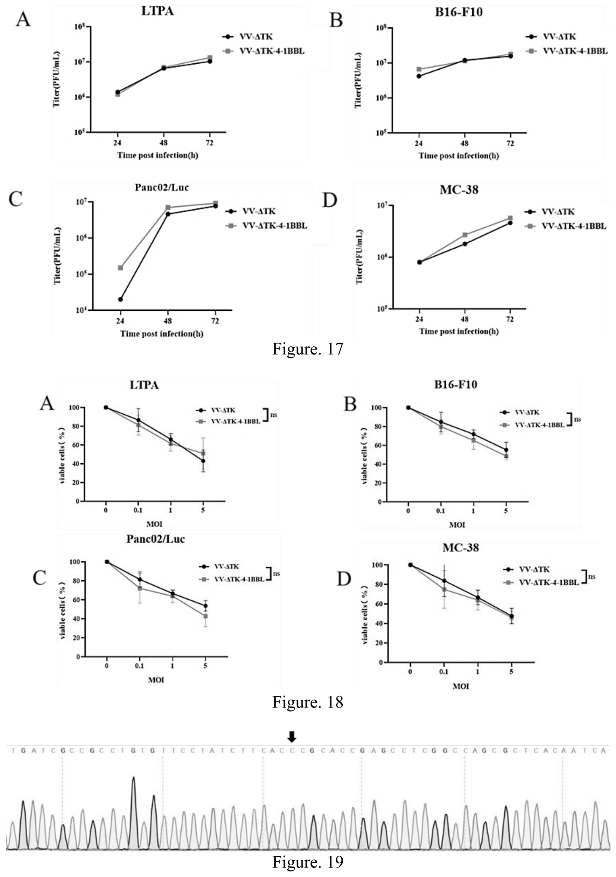

The results of the experiment (Fig. 17) showed that the recombination of the 4-1BBL gene into the vaccinia virus did not change the replication characteristics of the virus itself in tumor cells.

### V. Detecting and comparing the killing ability of VV-ΔTK-4-1BBL in tumor cells:

In order to confirm whether the gene recombination in this example has an effect on the oncolytic effect of the virus, LTPA cells, B16-F10 cells, Panc02/Luc cells and MC-38 cells were infected with each recombinant virus according to different MOIs (0, 0.1, 0.5 and 5), and the proportion of viable cells was detected by MTT assay after 48 h.

The results of the experiment (Fig. 18) showed that the oncolytic activity of the virus itself was not altered by the recombination of the 4-1BBL gene into the vaccinia virus.

### VI. Detection of synonymous mutation sites of 4-1BBL in the process of using a synonymously mutated 4-1BBL recombinant oncolytic vaccinia virus for tumor treatment:

In order to verify whether the synonymously mutated 4-1BBL recombinant oncolytic vaccinia virus can be detected in the process of tumor treatment, on the basis of the establishment of mouse intestinal cancer model, the experimental treatment with VV-ΔTK-4-1BBL was carried out, and then the tumor tissue was detected. The specific process is as follows.

C57BL/6 mice aged 8-12 weeks were inoculated intraperitoneally with MC-38 intestinal cancer cells at 5 ×10⁵/mouse. Nine days later, the mice were randomly divided into two groups, one group was intraperitoneally injected with VV ΔTK-4L according to 2 ×10⁸ PFU/mouse, and the other group was intraperitoneally injected with the same amount of PBS. After 48 h, the mice were killed and the tumor tissue was taken out. 10 mg of tumor tissue was dissolved in Trizol, and the total RNA was extracted and reversely transcribed into cDNA by PCR.

Primers were designed from the CDS region of the mouse 4-1BBL gene (NCBI Reference Sequence ID: NM_009404.3), and the primer sequences were as follows:
Pmf (SEQ ID No.19):
   5'-TATAAAGCTTATGGACCAGCACACACTTGATG-3';
Pmr (SEQ ID No.20):
   5'-TATATCTAGATCATTCCCATGGGTTGTCGG-3'.

The obtained cDNA was used as a template, Pmf and PmR were used as primers, a full-length fragment covering CDS of the mouse 4-1BBL gene was amplified by PCR, a PCR product was linked to a pUMc-T vector to transform competent DH5α E. coli, spread on an ampicillin-resistant LB plate, after formation of resistant colonies, single colonies were randomly selected for one-way sequencing. The sequencing primer sequence was 5'-GTTGTAAAACG ACGGCCAG-3' (SEQ ID No.21).

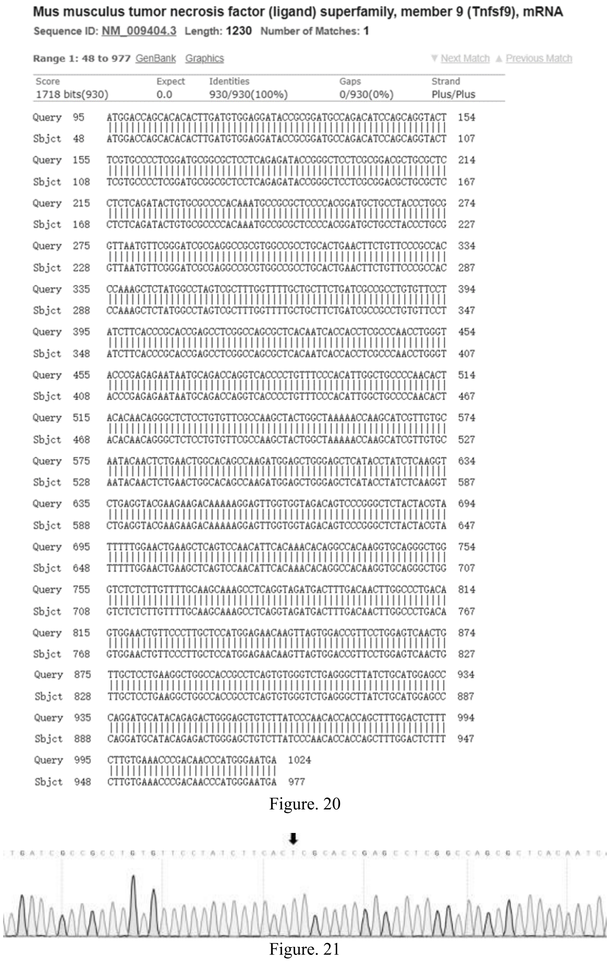

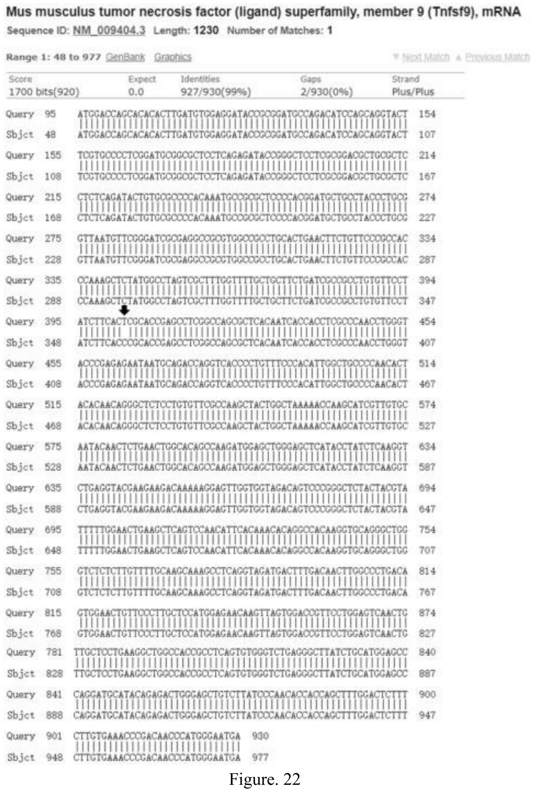

The experimental results showed that in the PBS treatment group, three clones are randomly selected for sequencing, and the obtained 4-1BBL fragments were all wild-type 4-1BBL, that is, 100% match with the CDS region of the mouse 4-1BBL gene (NCBI Reference Sequence ID: NM_009404.3) (Fig. 19 and Fig. 20). In Fig. 19, there is no mutation at the base C at position 309 in the CDS of the mouse 4-1BBL gene as indicated by the arrow; in the VV-ΔTK-4-1BBL group, 10 clones were randomly selected for sequencing, and the 4-1BBL sequence in one clone is 100% match with the CDS region of the mouse 4-1BBL gene (NCBI Reference Sequence ID: NM_009404.3). A base mutation from C to T at position 309 of the CDS was detected in all of the other 9 clones, while no mutation was detected at the other sites (Figs. 21 and 22). The arrow in Fig. 21 indicated that the base C at position 309 of the CDS of the mouse 4-1B BL gene was mutated to the base T. The wild-type 4-1BBL gene was derived from tumor-bearing mice, while a high proportion of the synonymously mutated 4-1BBL gene was derived from the oncolytic virus VV-ΔTK-4-1BBL.

The results proved that there was a background level of 4-1BBL gene expression in tumor cells, and also confirmed that during the in vivo treatment of VV-ΔTK-4-1BBL, a high level of synonymously mutated 4-1BBL gene was expressed by the rapid replication of virus after infection of tumor cells, and could be detected by molecular biology means.

### VII. Detecting the effect and mechanism of the synonymously mutated 4-1BBL recombinant oncolytic vaccinia virus on peripheral immune organs:

1. In order to investigate the anti-tumor effect and mechanism of VV-ΔTK-4-1BBL in vivo, and to verify the effect of VV-ΔTK-4-1BBL on peripheral immune tissues and organs, C57BL/6 mice aged 8-12 weeks were selected and inoculated with MC-38 intestinal cancer cells at 5 × 10⁵/mouse intraperitoneally. Nine days later, the mice were randomly divided into groups and injected intraperitoneally with VV-ΔTK-4L, VV-ΔTK and the same volume of PBS according to 2 ×10⁸ PFU/mouse. After 5 days, the mice were sacrificed, and the spleen of the mice was taken and weighed and the single cell suspension was prepared for counting.

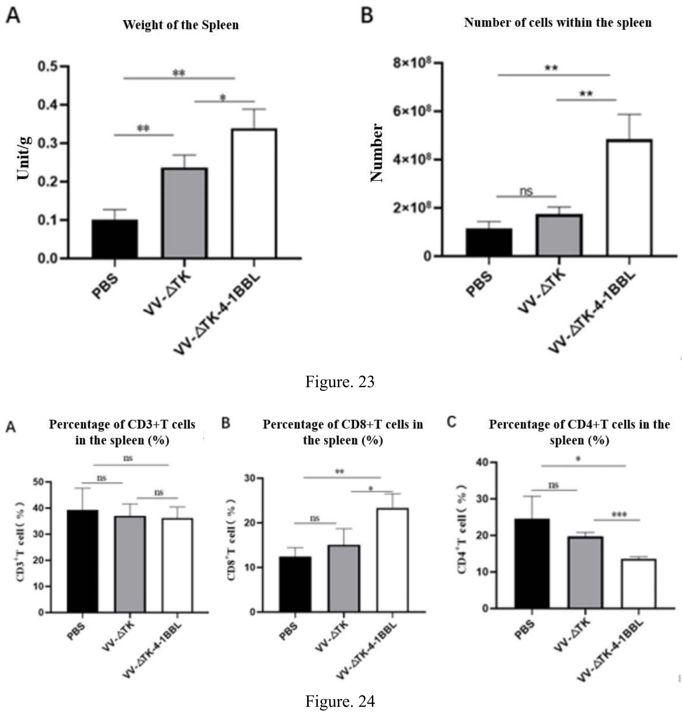

The results of the experiment (Fig. 23) showed that VV-ΔTK-4-1BBL treatment of MC-38 tumor-bearing mice resulted in an increase in the spleen weight (Fig. 23A) and the number of spleen cells (Fig. 23B), that is, it promoted the proliferation of peripheral immune cells and the hyperplasia of the peripheral immune tissues and organs.

The results of the experiment (Fig. 24) indicated that VV-ΔTK-4-1BBL treatment of MC-38 tumor-bearing mice promoted an increase in the proportion of CD3 + T cells (Fig. 24A), CD4 + T cells (Fig. 24B), and CD8 + T cells (Fig. 24C) in spleen cells. This confirmed that VV-ΔTK-4-1BBL could promote the proliferation of T cells and their subpopulations and NK cells in peripheral immune tissues and organs through the combined action of oncolytic virus and its expressed 4-1BBL molecule.

2. To investigate the anti-tumor effect and mechanism of VV-ΔTK-4-1BBL in vivo, and verify the effect of VV-ΔTK-4-1BBL on the expression of PD-1 and other immune checkpoint molecules of key cells of anti-tumor immunity, CD4 + T cells and CD8 + T cells, C57BL/6 mice aged 8-12 weeks were selected. MC-38 intestinal cancer cells were inoculated intraperitoneally at 5 × 10⁵/mouse. Nine days later, the mice were randomly divided into groups and injected intraperitoneally with VV-ΔTK-4L, VV-ΔTK and the same volume of PBS according to 2 ×10⁸ PFU/mouse. Five days later, the mice were sacrificed, and the spleens of the mice were taken to prepare a single cell suspension, which was analyzed by immunofluorescence staining and flow cytometry.

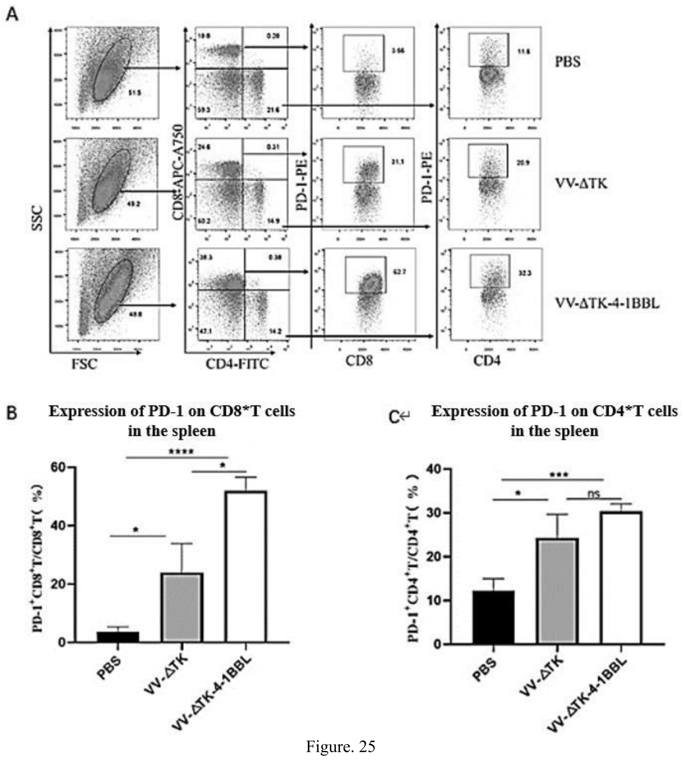

The experimental results (Fig. 25) showed that VV-ΔTK-4-1BBL can generate anti-tumor immune response after treating tumor, and can feedback and up-regulate the expression of PD-1 molecules on the surface of immune cells such as CD4 + T and CD8 + T. This result indicated that there is a necessary premise for the concept of VV-ΔTK-4-1BBL combined with PD-1/PD-L1 pathway blocker therapy to improve the anti-tumor efficacy.

### VIII. Detecting the effect and mechanism of the synonymously mutated 4-1BBL

### recombinant oncolytic vaccinia virus on the tumor microenvironment:

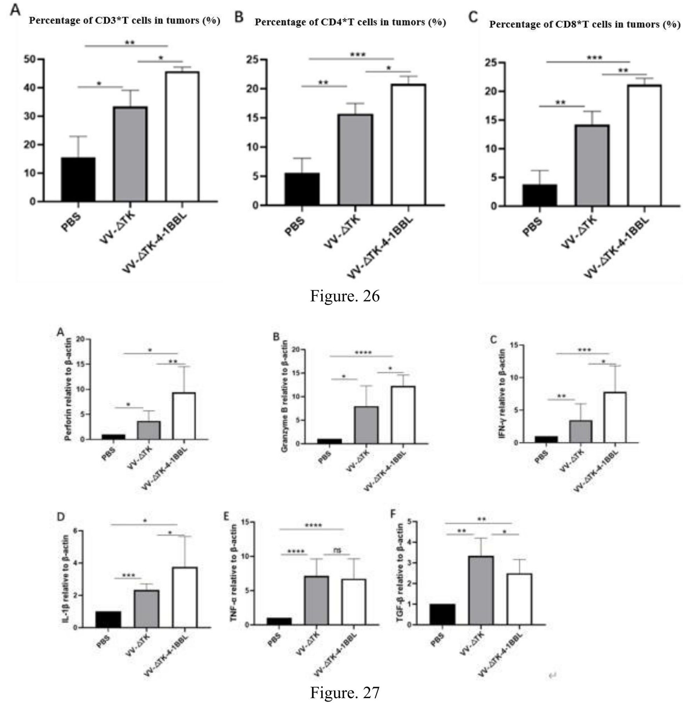

1. In order to verify the effect of VV-ΔTK-4-1BBL on the anti-tumor immune response in the tumor microenvironment, C57BL/6 mice aged 8-12 weeks were inoculated intraperitoneally with MC-38 intestinal cancer cells at a dose of 5 ×10⁵/mouse. Nine days later, the mice were randomly divided into groups and injected intraperitoneally with VV-ΔTK-4L, VV-ΔTK and the same volume of PBS according to 2 ×10⁸ PFU/mouse. Five days later, the mice were sacrificed, and the tumor tissue was taken to prepare a single cell suspension, which was analyzed by immunofluorescence staining and flow cytometry.
   The experimental results (Fig. 26) showed that the proportions of CD3 + T cells, CD8 + T cells and CD4 + T cells in the tumor tissues were significantly increased after the MC-38 tumor-bearing mice were treated with VV-ΔTK-4-1BBL. This indicated that VVΔTK-4-1BBL can promote an increase in the number of T cells and their subpopulations in tumor tissues and change the anti-tumor immune response through the combined action of oncolytic virus and its expressed 4-1BBL molecule.
2. In order to verify the effect of VV-ΔTK-4-1BBL on the anti-tumor immune response in the tumor microenvironment, C57BL/6 mice aged 8-12 weeks were inoculated intraperitoneally with MC-38 intestinal cancer cells at a dose of 5 ×10⁵/mouse. Nine days later, the mice were randomly divided into groups and injected intraperitoneally with VV-ΔTK-4L, VV-ΔTK and the same volume of PBS according to 2 ×10⁸ PFU/mouse. Five days later, the mice were sacrificed, and 50 mg of each tumor tissue was dissolved in Trizol to extract total RNA, which was reversely transcribed into cDNA and analyzed by Realtime PCR.

The experimental results (Fig. 27) showed that the anti-tumor immune molecules such as Perforin, Granzyme B, IFN-γ, IL-1β and TNF-α in the tumor tissues of the VV-ΔTK-4-1BBL treatment group were significantly increased. TGF-β and other negative immune molecules were significantly lower than those in the blank oncolytic virus control (VV-ΔTK) group. These results further proved that VV-ΔTK-4-1BBL can change the immune response in tumor tissues and promote the anti-tumor immune effect through the combined action of oncolytic virus and its expressed 4-1BBL molecule.

### IX. Verifying the anti-tumor effect of the synonymously mutated 4-1BBL recombinant oncolytic vaccinia virus in vivo:

In this example, the 4-1BBL gene was recombined into the vaccinia virus genome to construct a novel recombinant vaccinia virus. In order to compare their anti-tumor ability in vivo, the mouse intestinal cancer cell MC-38 was injected into the abdominal cavity of C57/BL6 mice to establish the abdominal tumor model of intestinal cancer. After 9 days, the mice were randomly divided into groups and injected with each recombinant virus in the abdominal cavity, and then the survival time of the mice was observed. The results showed that the survival time of tumor-bearing mice treated with VV-ΔTK (VV with TK gene knockout) and VV-ΔTK-4-1BBL (VV with TK gene knockout and 4-1BBL gene recombination) was longer than that treated with PBS treatment group, and the survival time of tumor-bearing mice treated with VV-ΔTK-4-1BBL was longer than that treated with VV-ΔTK.

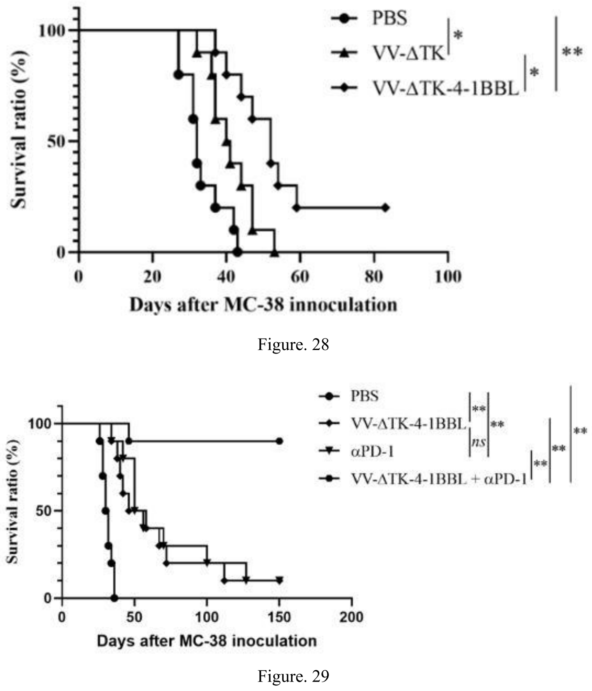

The experimental results (Fig. 28) showed that VV-ΔTK-4-1BBL can produce significant anti-tumor effects and prolong the survival time of the treated subjects when used for the treatment of tumors.

### X. Verifying the anti-tumor effect in vivo of the synonymously mutated 4-1BBL recombinant oncolytic vaccinia virus combined with PD-1 monoclonal antibody:

In order to verify the efficacy of VV-ΔTK-4-1BBL combined with immune checkpoint blockers, mouse intestinal cancer cell MC-38 was injected into the abdominal cavity of C57/BL6 mice to establish the abdominal tumor model of intestinal cancer, and the mice were randomly divided into groups after 9 days. The mice were divided into four groups according to PBS treatment group, VV-ΔTK-4-1BBL, PD-1 blocking monoclonal antibody treatment group and VV-ΔTK-4-1BBL combined with PD-1 blocking monoclonal antibody treatment group, and then the mice were injected intraperitoneally, and the survival time was observed.

The experimental results (Fig. 29) showed that the survival time of the tumor-bearing mice treated with VV-ΔTK-4-1BBL and PD-1 blocking monoclonal antibody was longer than that of the PBS treatment group, while the curative effects of the tumor-bearing mice treated with VV-ΔTK-4-1BBL and PD-1 blocking monoclonal antibody were equivalent. The therapeutic effect of VV-ΔTK-4-1BBL combined with PD-1 blocking monoclonal antibody was significantly better than that of VV-ΔTK-4-1BBL treatment group or PD-1 blocking monoclonal antibody treatment group.

This result proved that VV-ΔTK-4-1BBL can provide a powerful means to improve the efficacy of PD-1 monoclonal antibody and other immune checkpoint blockers in the treatment of tumors. The combination of VV-ΔTK-4-1BBL and immune checkpoint blockers can produce more obvious anti-tumor effect and improve the survival rate and survival time of the treated subjects.

The above description only recites preferred embodiments of the invention, and is not intended to limit the invention. Any modification, equivalent substitution or improvement within the spirit and principle of the invention shall be included in the protection scope of the invention.

## Claims

1. A recombinant oncolytic vaccinia virus, **characterized in that** the recombinant oncolytic vaccinia virus is operably inserted into a synonymously mutated exogenous gene capable of expressing 4-1BBL.

2. The recombinant oncolytic vaccinia virus according to claim 1, **characterized in that** the exogenous gene is inserted into TK gene of the vaccinia virus.

3. The recombinant oncolytic vaccinia virus of claim 1, **characterized in that** DNA sequence of the exogenous gene is shown as SEQ ID No.1 to SEQ ID No.3.

4. The recombinant oncolytic vaccinia virus according to claim 1, **characterized in that** amino acid sequence of the exogenous gene is shown as SEQ ID No.4.

5. The recombinant oncolytic vaccinia virus according to claim 1, **characterized in that** the exogenous gene is linked via an IRES sequence.

6. The recombinant oncolytic vaccinia virus according to claim 5, **characterized in that** the IRES sequence is shown as SEQ ID No.5.

7. A pharmaceutical composition, **characterized in that** the pharmaceutical composition comprises the recombinant oncolytic vaccinia virus according to any one of claims 1-6, a pharmaceutically acceptable carrier and a pharmaceutical excipient.

8. The pharmaceutical composition according to claim 7, **characterized in that** the administration mode of the pharmaceutical composition is direct injection and/or injection in combination with an endoscope; and the endoscope is preferably a laparoscope, a choledochoscope, a thoracoscope, an enteroscope, and a neuroendoscope.

9. Use of the recombinant oncolytic vaccinia virus according to any one of claims 1-6 or the pharmaceutical composition according to any one of claims 7-8 in the preparation of a drug for preventing or treating a tumor and/or a cancer.

10. The use according to claim 9, **characterized in that** the tumor and/or cancer is a solid tumor, preferably a cold tumor and/or a hot tumor, and the histological types thereof include, but are not limited to, pancreatic cancer, gallbladder cancer, liver cancer, colorectal cancer, gastric cancer, esophageal cancer, brain glioma, ovarian cancer, cervical cancer, prostate cancer, renal cancer, lung cancer, breast cancer, multiple myeloma, lymphoma, and melanoma.
